# EUROPEAN PATENT SPECIFICATION

(11) **EP 3 805 379 B1**
(45) Date of publication and mention of the grant of the patent: **02.12.2026**
(21) Application number: 19811465.4
(22) Date of filing: 28.05.2019
(51) Int. Cl.: C12N 9/04, C12P 21/02, C12N 15/53

(54) **ADH PROTEIN FAMILY MUTANT AND USE THEREOF**
ADH-PROTEINFAMILIE UND DEREN VERWENDUNG
MUTANT DE LA FAMILLE DES PROTÉINES ADH ET UTILISATION ASSOCIÉE

(30) Priority: 31.05.2018 CN 201810554584
(43) Date of publication of application: 14.04.2021
(73) Proprietor: Kangma-Healthcode (Shanghai) Biotech Co., Ltd, Pudong New Area Shanghai 201321 (CN)
(72) Inventor: GUO, Min, Shanghai 201321 (CN); DENG, Mini, Shanghai 201321 (CN); YU, Xue, Shanghai 201321 (CN)
(74) Representative: Cabinet Chaillot
(86) International application number: PCT/CN2019/088833
(87) International publication number: WO 2019/228353

(56) References cited:
- JP-A- 2013 146 236
- EHRIG TORSTEN ET AL: "General base catalysis in a glutamine for histidine mutant at position 51 of human liver alcohol dehydrogenase", BIOCHEMISTRY, vol. 30, no. 4, 1 January 1991 (1991-01-01), pages 1062 - 1068, XP055881839, ISSN: 0006-2960, DOI: 10.1021/bi00218a026
- JORNVALL HANS ET AL: "Human liver alcohol dehydrogenase: Amino acid substitution in the [beta]2[beta]2 Oriental isozyme explains functional properties, establishes an active site structure, and parallels mutational exchanges in the yeast enzyme", vol. 81, 1 May 1984 (1984-05-01), pages 3024 - 3028, XP055881831, Retrieved from the Internet <URL:https://www.pnas.org/content/pnas/81/10/3024.full.pdf>
- WILLS CHRISTOPHER ET AL: "Amino acid substitutions in two functional mutants of yeast alcohol dehydrogenase", vol. 279, 21 June 1979 (1979-06-21), pages 734 - 736, XP055881900, Retrieved from the Internet <URL:https://www.nature.com/articles/279734a0.pdf>
- SAKODA H ET AL: "Cloning and sequencing of the gene coding for alcohol dehydrogenase of Bacillus stearothermophilus and rational shift of the optimum pH", JOURNAL OF BACTERIOLOGY (PRINT), vol. 174, no. 4, 1 February 1992 (1992-02-01), US, pages 1397 - 1402, XP055881865, ISSN: 0021-9193, DOI: 10.1128/jb.174.4.1397-1402.1992
- YONG: "Optimized expression in Pichia pastoris eliminates common pro tein contaminants from subsequent His-tag purification", BIOTECHNOLOGY LETTERS, 10 December 2013 (2013-12-10), XP055659813, ISSN: 1573-6776
- DATABASE GenBank 1 January 2015 (2015-01-01), "unnamed protein product [Kluyveromyces dobzhanskii CBS 2104]H", Database accession no. CD 096326. 1

## Description

### BACKGROUND OF THE INVENTION

### 1. Technical Field

The present invention relates to the field of biotechnology, and more particularly, to ADH protein family mutants and use thereof.

### 2. Description of the Related Art

Protein separation and purification refers to processes in which target proteins are purified from mixtures, extracts, cell lysis liquid, reaction products and the like by fusing poly-tag at one end of the target proteins or via the specific characteristics of the target proteins and by using chromatography method to separate other substances. Affinity chromatography refers to a method that one of two molecules having affinity is fixed on an insoluble matrix, and the other molecule is separated and purified based on the specificity and reversibility of the affinity between the two molecules. Common poly-tags used for affinity chromatography mainly include histidine (His), Glutathione S-transferase (GST), Maltose Binding Protein (MBP), etc. The principle of immobilized metal-chelating affinity chromatography (IMAC) is mainly based on the fact that amino acid residues on protein surface can form different affinity with metal ions, which can be divided into three types: electrostatic attraction, covalent binding and coordination bond binding. When protein surface contains histidine, cysteine or tryptophan, the corresponding imidazolyl group, thiol group or indolyl group can form coordination bond with metal ion. This method has become a commonly used protein purification tool by virtue of convenient expression, low cost, small impact on the properties of the target proteins and other advantages.

However, in practice, this purification method also has certain problems. Take Ni-NTA resin for example, some non-target proteins also have several discontinuous histidine residues on the surface of their three-dimensional structure, which results in that those non-target proteins also bind to Ni-NTA resin to a certain extent, thereby interfering with the purity of the final target protein.

Yong Chen and colleagues (Optimized expression in Pichia pastoris eliminates common protein contaminants from subsequent His-tag purification (Biotechnol Letter 2014, 36:711-718) describe knocking-out mitochondrial alcohol dehydrogenase isozyme III (mADH) in order to eliminate the predominant contaminant, mADH, in IMAC. They describe surface His residues of ADH involved in binding to the IMAC chromatography resin.

Accordingly, there is an urgent need in the art to invent a method for engineering the histidine residues of non-target proteins to make them unable to bind to Ni ion affinity medium, such as Ni-NTA resin, so as to achieve the effect of improving the purity of target proteins.

### SUMMARY OF THE INVENTION

One object of the present invention is to provide a method for engineering histidine residues of non-target proteins to make them unable to bind to Ni ion affinity medium, such as Ni-NTA resin, so as to increase the purity of target proteins.

The present invention provides a cell-free *in vitro* protein translation system or transcription and translation system comprising a yeast cell lysate of a genetically engineered cell strain, wherein gene encoding its endogenous ADH protein is mutated to a polynucleotide encoding an alcohol dehydrogenase (ADH) mutant protein, so that the mutant protein has ADH activity, and compared with the ADH protein in wild-type *Kluyveromyces lactis,* the mutant protein has reduced binding ability to Ni medium.

The present invention also provides a use of the cell-free in vitro protein translation system or transcription and translation system for in vitro protein synthesis, where improved product purity of expressed protein is provided when using Ni medium for purification compared with that of obtained from the wild-type strain.

According to a first aspect not part of the claimed invention, an alcohol dehydrogenase (ADH) mutant protein is described. The mutant protein is a non-natural protein, and the mutant protein has mutations of at least one histidine (H) among amino acids 45-149 of wild-type alcohol dehydrogenase (ADH).

In another preferred embodiment, the mutation does not include histidine (H) at positions 67, 69, 93 and / or 94 of the wild-type alcohol dehydrogenase (ADH).

In another preferred embodiment, compared with the wild-type alcohol dehydrogenase (ADH), the binding ability of the alcohol dehydrogenase (ADH) mutant protein to Ni medium is reduced by 10%, preferably, by 25%, more preferably, by 50%, more preferably, by 80%, and most preferably, by 100%.

In another preferred embodiment, the histidines can be independently mutated to a basic amino acid.

In another preferred embodiment, the types of independent mutations of the histidine can be the same or different.

In another preferred embodiment, the histidines can be independently mutated to one or more amino acids selected from the following group: lysine (K), asparagine (N), glutamine (Q), arginine (R) and combinations thereof.

In another preferred embodiment, the mutant protein is derived from *K. lactis.*

In another preferred embodiment, the alcohol dehydrogenase (ADH) includes ADH1, ADH2, ADH3 and/or ADH4 protein.

In another preferred embodiment, the mutant protein has mutations at one or more core amino acids of wild-type alcohol dehydrogenase (ADH1) corresponding to SEQ ID NO.: 1, wherein the one or more core amino acids are selected from the following group:
histidine (H) at position 47;
histidine (H) at position 51; and
histidine (H) at position 124.

In another preferred embodiment, the mutant protein has mutations at one or more core amino acids of wild-type alcohol dehydrogenase (ADH2) corresponding to SEQ ID NO.: 26, wherein the one or more core amino acids are selected from the following group:
histidine (H) at position 45;
histidine (H) at position 49; and
histidine (H) at position 122.

In another preferred embodiment, the mutant protein has mutations at one or more core amino acids of wild-type alcohol dehydrogenase (ADH3) corresponding to SEQ ID NO.: 27, wherein the one or more core amino acids are selected from the following group:
histidine (H) at position 71;
histidine (H) at position 75; and
histidine (H) at position 148.

In another preferred embodiment, the mutant protein has mutations at one or more core amino acids of wild-type alcohol dehydrogenase (ADH4) corresponding to SEQ ID NO.: 28, wherein the one or more core amino acids are selected from the following group:
histidine (H) at position 72;
histidine (H) at position 76; and
histidine (H) at position 149.

In another preferred embodiment, the histidine (H) at position 47 is mutated to one or more amino acids selected from the following group: lysine (K), asparagine (N), glutamine (Q), arginine (R) and combinations thereof, preferably, lysine (K), asparagine (N) and/or arginine (R), more preferably, lysine (K) and asparagine (N).

In another preferred embodiment, the histidine (H) at position 51 is mutated to one or more amino acids selected from the following group: lysine (K), arginine (R) and a combination thereof, preferably, lysine (K) and/or arginine (R), more preferably, arginine (R).

In another preferred embodiment, the histidine (H) at position 124 is mutated to one or more amino acids selected from the following group: lysine (K), arginine (R) and a combination thereof, preferably, lysine (K).

In another preferred embodiment, the mutant protein has mutations at one or more core amino acids of wild-type alcohol dehydrogenase (ADH) corresponding to SEQ ID NO.: 1, wherein the one or more core amino acids are selected from the following group:
histidine (H) at position 47; and
histidine (H) at position 124.

In another preferred embodiment, the histidine (H) at position 45 is mutated to one or more amino acids selected from the following group: lysine (K), arginine (R), asparagine (N), glutamine (Q) and combinations thereof.

In another preferred embodiment, histidine (H) at position 49 is mutated to one or more amino acids selected from the group consisting of lysine (K), arginine (R) and a combination thereof.

In another preferred embodiment, histidine (H) at position 122 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 26, wherein the one or more core amino acids are selected from the following group:
histidine (H) at position 122; and
histidine (H) at position 45.

In another preferred embodiment, the histidine (H) at position 71 is mutated to one or more amino acids selected from the following group: lysine (K), arginine (R), asparagine (N), glutamine (Q) and combinations thereof.

In another preferred embodiment, the histidine (H) at position 75 is mutated to one or more amino acids selected from the following group: lysine (K), arginine (R) and a combination thereof.

In another preferred embodiment, histidine (H) at position 148 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 27, wherein the one or more core amino acids are selected from the following group:
histidine (H) at position 148; and
histidine (H) at position 71.

In another preferred embodiment, the histidine (H) at position 72 is mutated to one or more amino acids selected from the following group: lysine (K), arginine (R), asparagine (N), glutamine (Q) and combinations thereof.

In another preferred embodiment, the histidine (H) at position 76 is mutated to one or more amino acids selected from the following group: lysine (K), arginine (R) and a combination thereof.

In another preferred embodiment, the histidine (H) at position 149 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 28, wherein the one or more core amino acids are selected from the following group:
histidine (H) at position 149; and
histidine (H) at position 72.

In another preferred embodiment, the mutation is selected from the following group: H47K; H47N; H47Q; H47R; H51K; H51R; H124K; H124R; H47K and H124K; H47N and H124K; H47Q and H124K; H47R and H124K.

In another preferred embodiment, the mutation is selected from the following group: H47K; H47N; H51R; H124R; H47K and H124K; H47N and H124K.

In another preferred embodiment, the mutation is selected from the following group: H122K; H122R; H45K; H45R; H45N; H45Q; H49K; H49R; H122K and H45K; H122K and H45R; H122K and H45N; H122K and H45Q.

In another preferred embodiment, the mutation is selected from the following group: H148K; H148R; H71K; H71R; H71N; H71Q; H75K; H75R; H148K and H71K; H148K and H71R; H148K and H71N; H148K and H71Q.

In another preferred embodiment, the mutation is selected from the following group: H149K; H149R; H72K; H72R; H72N; H72Q; H76K; H76R; H149K and H72K; H149K and H72R; H149K and H72N; H149K and H72Q.

In another preferred embodiment, the amino acid sequence of the alcohol dehydrogenase (ADH) mutant protein is selected from the following group:
(1) a polypeptide whose amino acid sequence is any one of SEQ ID NO.: 2-13.
(2) a polypeptide derived from the polypeptides of amino acid sequences shown in SEQ ID NO.: 2-13, and having the function of the polypeptide described in (1), wherein the polypeptide is formed by substitution, deletion or addition of one or several (preferably 1-20, more preferably 1-15, more preferably 1-10, more preferably 1-8, more preferably 1-3, most preferably 1) amino acid residues in the amino acid sequence shown in any one of SEQ ID NO.: 2-13.

In another preferred embodiment, the amino acid sequence of the mutant protein is any one of SEQ ID NO.: 2-13.

In another preferred embodiment, the amino acid sequence of the mutant protein has at least 70% (preferably at least 75%, 80%, 85%, 90%, and more preferably at least 95%%, 96%, 97%, 98%, 99%) identity with the sequence SEQ ID NO.: 2-13.

In another preferred embodiment, except for the mutation (e.g., positions 47, 51 and/or 124), the remaining amino acid sequence of the mutant protein is identical or substantially identical to the sequence shown in SEQ ID NO.: 1.

In another preferred embodiment, except for the mutation (e.g., positions 45, 49 and/or 122), the remaining amino acid sequence of the mutant protein is identical or substantially identical to the sequence shown in SEQ ID NO.: 26.

In another preferred embodiment, except for the mutation (e.g., positions 71, 75 and/or 148), the remaining amino acid sequence of the mutant protein is identical or substantially identical to the sequence shown in SEQ ID NO.: 27.

In another preferred embodiment, except for the mutation (e.g., positions 72, 76 and/or 149), the remaining amino acid sequence of the mutant protein is identical or substantially identical to the sequence shown in SEQ ID NO.: 28.

In another preferred embodiment, that "the remaining amino acid sequence is substantially identical" refers to up to 50 (preferably 1-20, more preferably 1-10, more preferably 1-5) amino acids are different, wherein, the difference includes substitution, deletion or addition of amino acids, and the mutant protein still can (i) improve the expression purity, efficiency, and/or yield of the exogenous protein in an *in-vitro* cell-free synthesis system; and/or (ii) reduce the binding ability of the mutant protein to Ni medium.

In another preferred embodiment, the homology with the sequence shown in SEQ ID NO.: 1, 26, 27 or 28 is at least 80%, preferably at least 85% or 90%, more preferably at least 95%, most preferably at least 98% or 99%.

According to a second aspect not part of the claimed invention, a polynucleotide is described, wherein the polynucleotide encodes the mutant protein of the first aspect.

In another preferred embodiment, the polynucleotide is selected from the following group:
(a) a polynucleotide encoding the polypeptide shown in any one of SEQ ID NO.: 2-13;
(b) a polynucleotide whose sequence is any one of SEQ ID NO.: 14-25;
(c) a polynucleotide encoding the polypeptide shown in any one of SEQ ID NO.: 2-13; the homology of the sequence of the polynucleotide to the sequence shown in the nucleotide sequence of the wild-type ADH1 protein is ≥95% (preferably ≥98%);
(d) a polynucleotide complementary to any of the polynucleotides described in (a) to (c).

In another preferred embodiment, the polynucleotide additionally contains accessory elements in the flank of the ORF of the mutant protein, wherein the accessory elements are selected from the following group: a signal peptide, a secretory peptide, a tag sequence (such as 6His) and combinations thereof.

In another preferred embodiment, the polynucleotide is selected from the following group: a DNA sequence, a RNA sequence and a combination thereof.

According to a third aspect not part of the claimed invention, a vector is described ; wherein, the vector contains the polynucleotide according to the second aspect.

In another preferred embodiment, the vector includes one or more promoters; wherein, the promoters are operably linked to the nucleic acid sequence, enhancer, transcription termination signal, polyadenylation sequence, origin of replication, selectable marker, nucleic acid restriction site and/or homologous recombination site.

In another preferred embodiment, the vector includes a plasmid and a viral vector.

In another preferred embodiment, the viral vector is selected from the following group: adeno-associated virus (AAV), adenovirus, lentivirus, retrovirus, herpes virus, SV40, poxvirus and combinations thereof.

In another preferred embodiment, the vector includes an expression vector, a shuttle vector and an integration vector.

According to a fourth aspect not part of the claimed invention a host cell is described ; wherein, the host cell contains the vector according to the third aspect, or the host cell has the polynucleotide according to the second aspect integrated in its genome.

In another preferred embodiment, the host cell is a eukaryotic cell, such as a yeast cell, a plant cell or a mammal cell (including human and non-human mammal cells).

In another preferred embodiment, the host cell is a prokaryotic cell, such as *E. coli.*

In another preferred embodiment, the yeast cell is selected from one or more yeasts from the following group: *Pichia pastoris, Kluyveromyces* and a combination thereof; preferably, the yeast cell includes: *Kluyveromyces,* more preferably *Kluyveromyces marxianus* and/or *Kluyveromyces lactis.*

In another preferred embodiment, the host cell is selected from the following group: *E. coli,* wheat germ cell, insect cell, SF9 cell, Hela cell, HEK293 cell, CHO cell, yeast cell and combinations thereof.

According to a fifth aspect, the present invention provides a cell-free in vitro protein translation or transcription and translation system according to claim 1.

According to a sixth aspect not part of the claimed invention, an enzyme preparation is described, wherein the enzyme preparation comprises the alcohol dehydrogenase (ADH) mutant protein according to the first aspect.

In another preferred embodiment, the enzyme preparation includes an injection and/or a lyophilized preparation.

According to a seventh aspect, the present invention provides a use of the system as claimed in claim 2.

In another preferred embodiment, the Ni is selected from the following group: nickel element, nickel ion, free nickel ion, chromatography medium with nickel being bound, Ni²⁺, Ni-beads, Ni-NTA and combinations thereof.

It should be understood that, within the scope of the present invention, above-mentioned technical features and technical features specifically described hereinafter (e.g., embodiments or examples) can be combined with each other, whereby forming new or preferred technical solutions. For the simplicity, details will not be repeated herein.

### BRIEF DESCRIPTION OF THE DRAWINGS

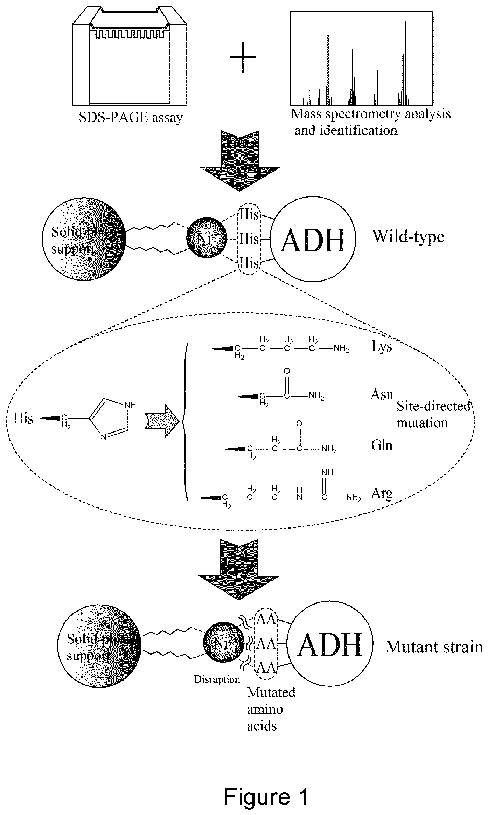

Figure 1 shows a design route for engineering the gene of ADH1 in the *K. lactis* genome to eliminate its binding to Ni medium.
Figure 2 shows a sequence homology comparison of the *K1*ADH family protein ADH1-4. The black region represents the sequences have high homology.
Figure 3 shows a sequence homology comparison between *Kl*ADH1 and *Sc*ADH1, wherein the sequence homology between the two is 84.9%.

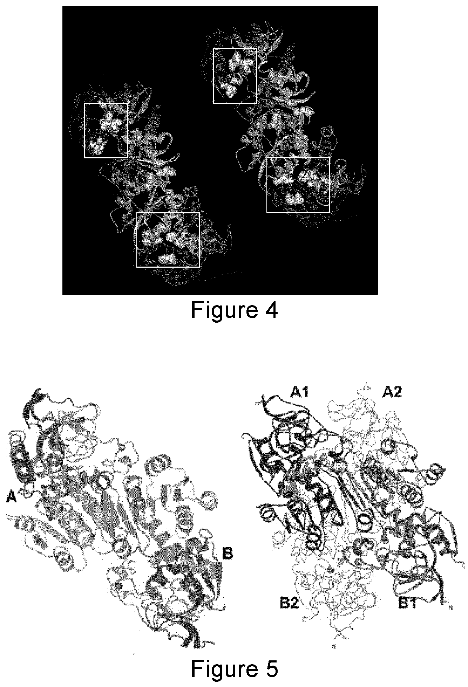

Figure 4 shows a homology modeling of *Kl*ADH4 with 2HCY as a template. Both 2HCY and *Kl*ADH4 are tetramers, and the six-His aggregation region of each monomer is represented by boxes, respectively.
Figure 5 shows a structure diagram of ScADH1 tetramer. The two subunits A and B form a dimer through the NADH/NAD⁺ binding domain, and two AB dimers form a "back-to-back" tetramer.
Figure 6 shows a diagram of the amino acid structure around H66 in *Sc*ADH1. H66 is involved in chelating the catalytic Zn²⁺ in two conformations of ADH1. Figure A shows a closed conformation with NAD being bound, and Figure B shows an open conformation with no NAD being bound.
Figure 7 shows a diagram of the amino acid structure around H48 in *Sc*ADH1. H48 is involved in the formation of a ternary complex and affects the proton transfer process of the substrate.
Figure 8 shows a diagram of the amino acid structure around H44 in *Sc*ADH1. ND1 on the imidazole ring of H44 may be involved in the binding of NAD⁺/NADH.

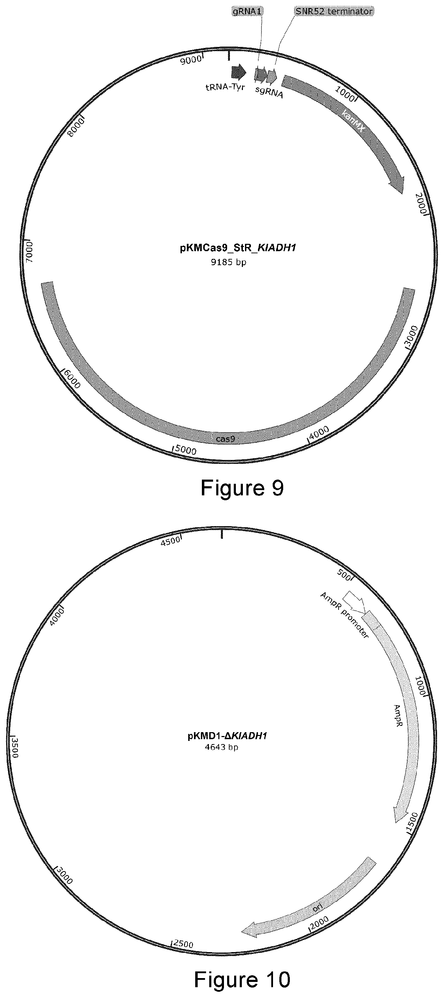

Figure 9 shows a plasmid profile of pKMCas9_StR_*KlADH1*. The plasmid has a tRNA-Tyr promoter, a SNR52 terminator and a kana selection marker.
Figure 10 shows a plasmid profile of pKMD1-Δ*KlADH1*. Homologous arm 1 and homologous arm 2 are the gene sequences at about 800 bp upstream and downstream of the ORF of the *KlADH1* gene, respectively, and the plasmid has an Amp selection marker.

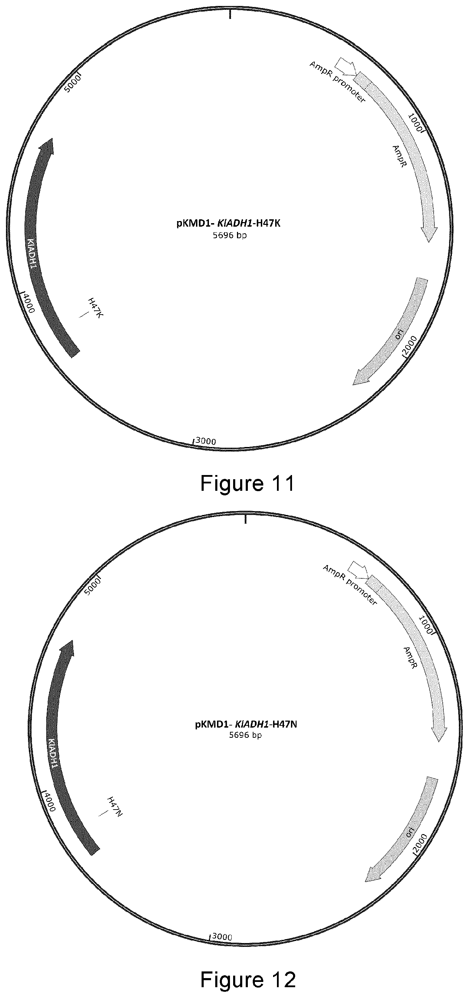

Figure 11 shows a plasmid profile of pKMD1-*KlADH1*-H47K. Homologous arm 1 and homologous arm 2 are the gene sequences at about 800 bp upstream and downstream of the ORF of the *KlADH1* gene, respectively, and the plasmid has an Amp selection marker.
Figure 12 shows a plasmid profile of pKMD1-*KlADH1*-H47N. Homologous arm 1 and homologous arm 2 are the gene sequences at about 800 bp upstream and downstream of the ORF of the *KlADH1* gene, respectively, and the plasmid has an Amp selection marker.

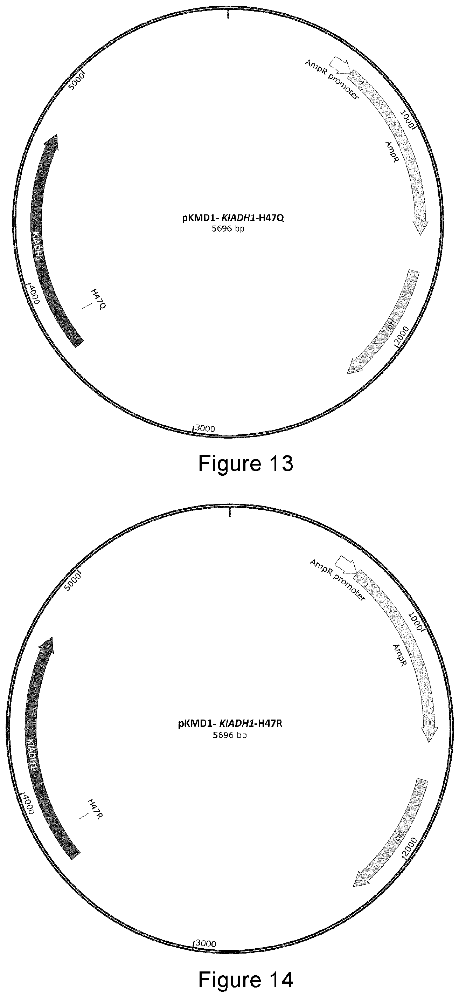

Figure 13 shows a plasmid profile of pKMD1-*KlADH1*-H47Q. Homologous arm 1 and homologous arm 2 are the gene sequences at about 800 bp upstream and downstream of the ORF of the *KlADH1* gene, respectively, and the plasmid has an Amp selection marker.
Figure 14 shows a plasmid profile of pKMD1-*KlADH1*-H47R. Homologous arm 1 and homologous arm 2 are the gene sequences at about 800 bp upstream and downstream of the ORF of the *KlADH1* gene, respectively, and the plasmid has an Amp selection marker.

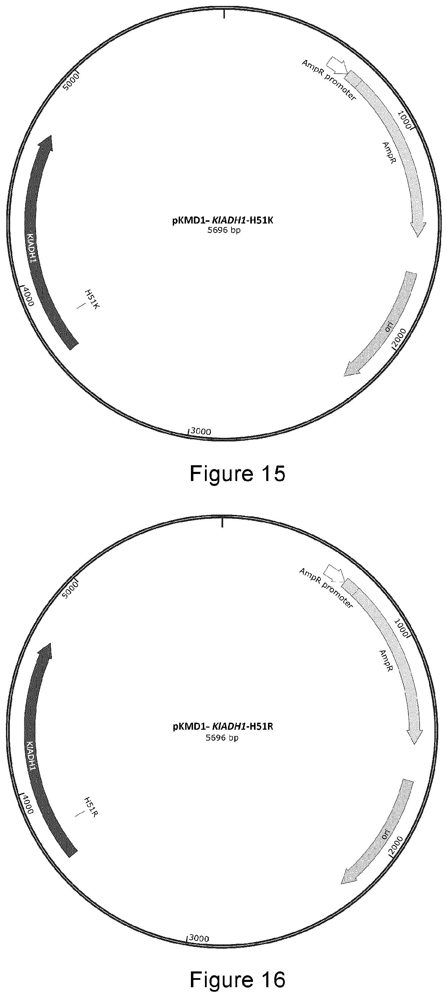

Figure 15 shows a plasmid profile of pKMD1-*KlADH1*-H51K. Homologous arm 1 and homologous arm 2 are the gene sequences at about 800 bp upstream and downstream of the ORF of the *KlADH1* gene, respectively, and the plasmid has an Amp selection marker.
Figure 16 shows a plasmid profile of pKMD1-*KlADH1*-H51R. Homologous arm 1 and homologous arm 2 are the gene sequences at about 800 bp upstream and downstream of the ORF of the *KlADH1* gene, respectively, and the plasmid has an Amp selection marker.

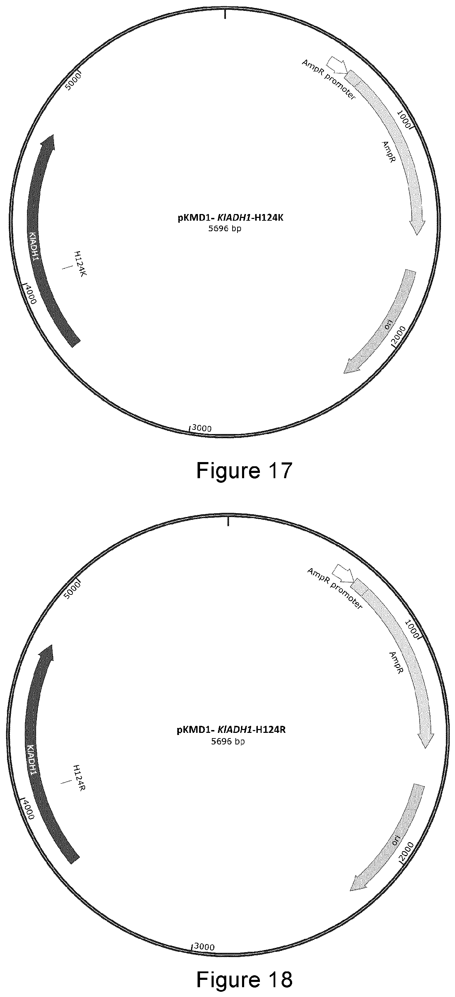

Figure 17 shows a plasmid profile of pKMD1-*KlADH1*-H124K. Homologous arm 1 and homologous arm 2 are the gene sequences at about 800 bp upstream and downstream of the ORF of the *KlADH1* gene, respectively, and the plasmid has an Amp selection marker.
Figure 18 shows a plasmid profile of pKMD1-*KlADH1*-H124R. Homologous arm 1 and homologous arm 2 are the gene sequences at about 800 bp upstream and downstream of the ORF of the *KlADH1* gene, respectively, and the plasmid has an Amp selection marker.

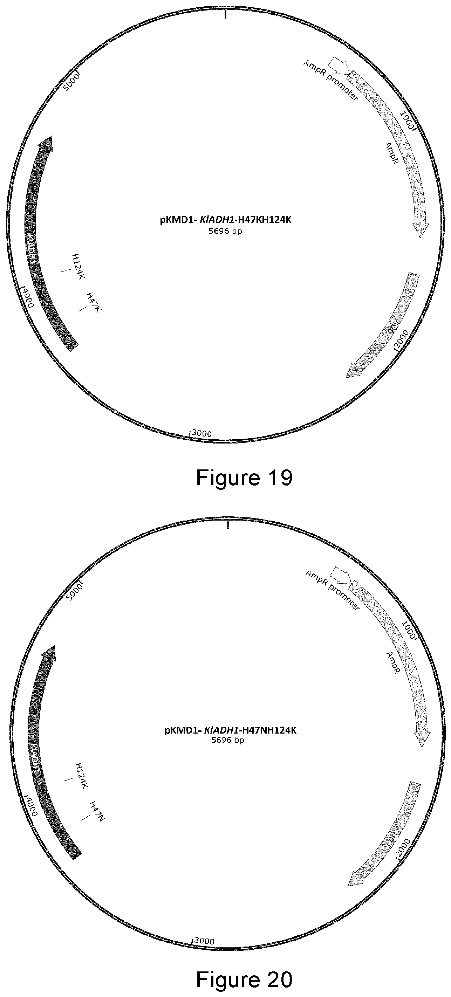

Figure 19 shows a plasmid profile of pKMD1-*KlADH1*-H47KH124K. Homologous arm 1 and homologous arm 2 are the gene sequences at about 800 bp upstream and downstream of the ORF of the *KlADH1* gene, respectively, and the plasmid has an Amp selection marker.
Figure 20 shows a plasmid profile of pKMD1-*KlADH1*-H47NH124K. Homologous arm 1 and homologous arm 2 are the gene sequences at about 800 bp upstream and downstream of the ORF of the *KlADH1* gene, respectively, and the plasmid has an Amp selection marker.

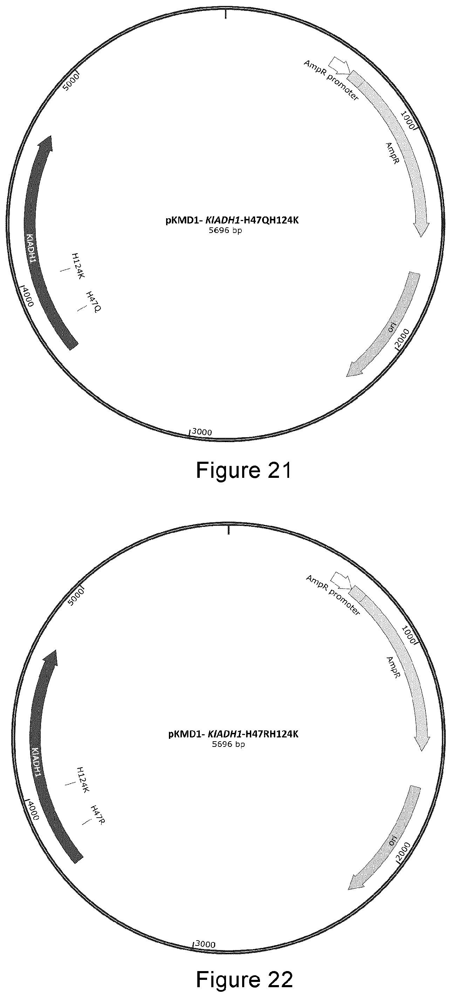

Figure 21 shows a plasmid profile of pKMD1-*KlADH1*-H47QH124K. Homologous arm 1 and homologous arm 2 are the gene sequences at about 800 bp upstream and downstream of the ORF of the *KlADH1* gene, respectively, and the plasmid has an Amp selection marker.
Figure 22 shows a plasmid profile of pKMD1-*KlADH1*-H47RH124K. Homologous arm 1 and homologous arm 2 are the gene sequences at about 800 bp upstream and downstream of the ORF of the *KlADH1* gene, respectively, and the plasmid has an Amp selection marker.
Figure 23 shows a polyacrylamide gel analysis purified by Ni-NTA. As shown in the figure, knockout of *KlADH1* gene and His mutation at different positions can significantly reduce the affinity of *Kl*ADH1 to Ni-NTA. The size of the target band is about 37KD, and the bands from left to right refer to marker, *K. lactis* wild-type cell strain WT, cell strain ΔADH1 with *Kl*ADH1 being knocked-out, *Kl*ADH1 single mutant cell strain H47K, H47N, H47R, H51K, H124K, H124KH47K and H124KH47N, respectively.
Figure 24 shows an analysis of the cell-free transcription and translation activity of wild-type cell strain WT, cell strain ΔADH1 with *Kl*ADH1 being knocked-out, and cell strain H47K with single mutation of *Kl*ADH1. As shown in the figure, the bands from left to right refer to ΔADH1, *Kl*ADH1 H47K, *K. lactis* wild-type cell strain WT and negative control, respectively.

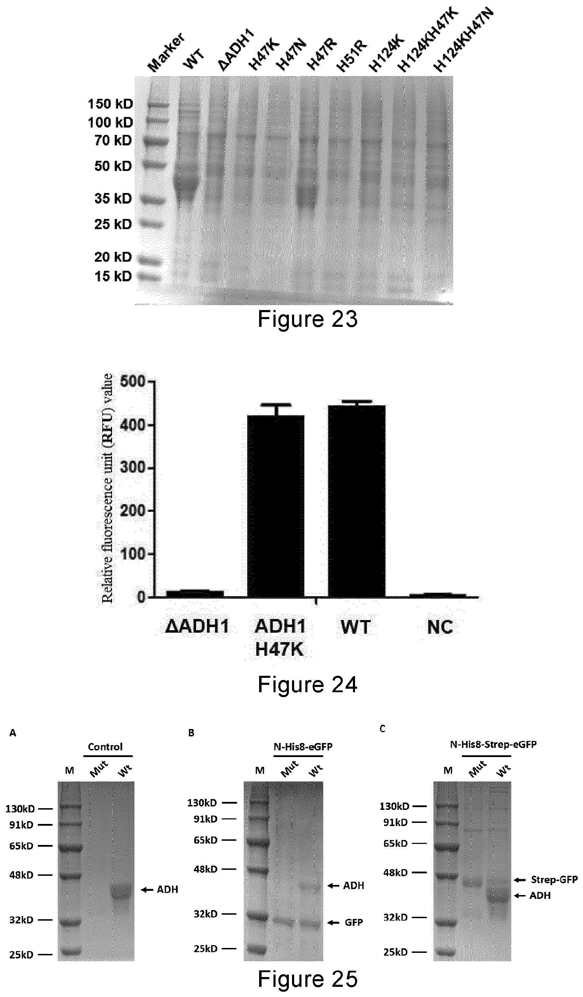

Figure 25 shows a purification analysis of the target protein prepared by cell-free transcription and translation of wild-type cell strain WT and cell strain H47K (Mut) with single mutation of *Kl*ADH1. As shown in the figure, the bands from left to right refer to cell strain H47K (Mut) with single mutation of *Kl*ADH1 and *K. lactis* wild-type cell strain WT, respectively. Figure A shows the affinity analysis of between *Kl*ADH1 and Ni-NTA without the addition of exogenous target proteins. Figure B shows the purification analysis of eGFP when the target protein N-His8-eGFP was added to the cell-free transcription and translation system of cell strain H47K with single mutation of *Kl*ADH1. Figure C shows the purification analysis of Strep-eGFP when the target protein N-His8-Strep-eGFP was added to the cell-free transcription and translation system of cell strain H47K with single mutation of *Kl*ADH1.

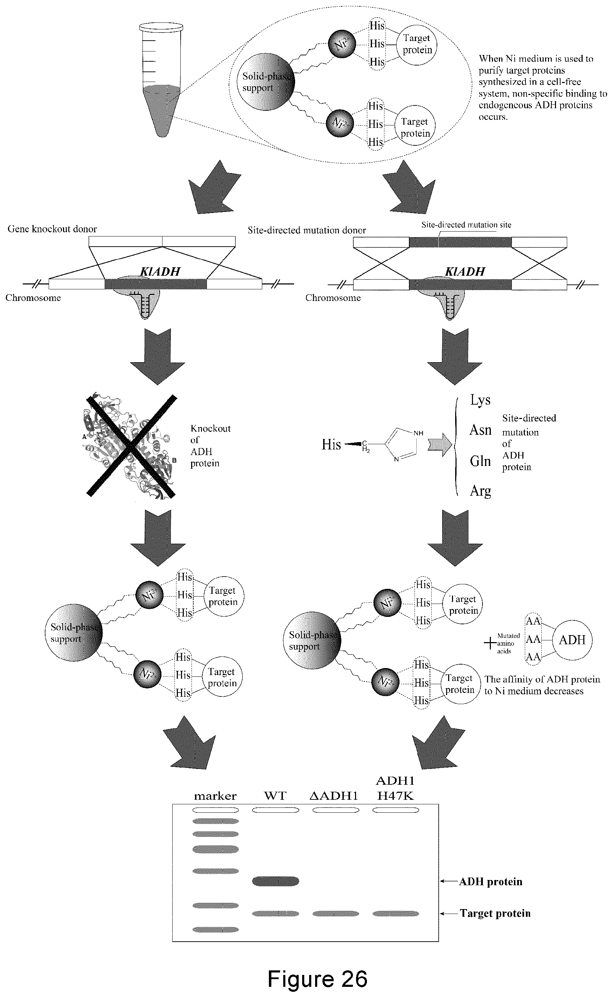

Figure 26 summarizes elimination of the binding ability of *Kl*ADH1 to Ni medium in order to improve the purity and specificity of the target protein by knockout of *KlADH1* gene and site-directed mutation to *KlADH1* gene, respectively.

### DETAILED DESCRIPTION

After extensive and in-depth research, the inventors unexpectedly obtained an ADH family protein mutant. Compared with the wild-type ADH family protein, the ADH family protein mutant can significantly (i) improve the expression purity, efficiency, and/or yield of the exogenous protein in an *in-vitro* cell-free synthesis system; and/or (ii) reduce the binding ability of the mutant protein to Ni medium. On this basis, the inventors completed the present invention.

### TERMS

In order to facilitate the understanding of this disclosure, certain terms are first defined. As used in this application, unless otherwise explicitly stated herein, each of the following terms shall have the meaning given below. Other definitions are stated throughout the application.

The term "about" may refer to a value or composition within an acceptable error range of a particular value or composition determined by those of ordinary skill in the art, which will depend in part on how the value or composition is measured or determined. For example, as used herein, the expression "about 100" includes all values between 99 and 101 (e.g., 99.1, 99.2, 99.3, 99.4, etc.).

As used herein, the terms "contain" or "include" can be understood to be open-ended, semi-closed-ended, or closed-ended. In other words, the terms also include "consist substantially of" or "consist of".

Sequence identity (or homology) is determined by comparing two aligned sequences along a predetermined comparison window (which can be 50%, 60%, 70%, 80%, 90%, 95% or 100% of the length of the reference nucleotide sequence or protein) and determining the number of locations where the same residue appears. Normally, this is expressed as a percentage. The measurement of nucleotide sequence identity is a method well known to those skilled in the art.

As used herein, the terms "subject" and "subject in need" refer to any mammal or non-mammal. The mammal includes, but is not limited to, human, vertebrate (such as rodents), non-human primate, cow, horse, dog, cat, pig, sheep, goat, giraffe, deer, camel, antelope, hare and rabbit.

### ADH FAMILY PROTEINS

A large amount of alcohol dehydrogenase (ADH) family proteins can be found in human and animal livers, plants and microbial cells. As a key enzyme for the metabolism of short-chain alcohols in organisms, it plays an important role in many physiological processes. For example, in humans and mammals, alcohol dehydrogenase and acetaldehyde dehydrogenase (ALDH) constitute the alcohol dehydrogenase system, which participates in the metabolism of alcohol in the body.

The ADH family proteins include ADH1, ADH2, ADH3 and/or ADH4 proteins.

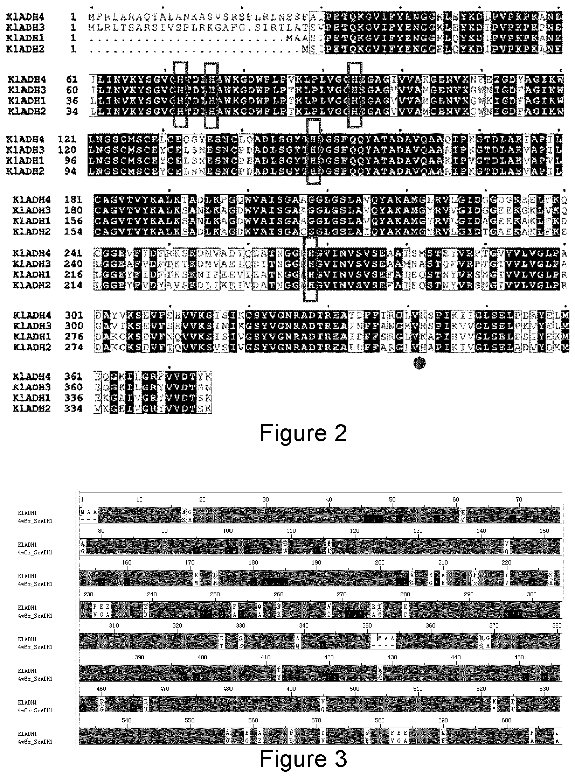

As shown in Figure 2, the present invention found for the first time that ADH2 (SEQ ID NO.: 26, positions 45-122), ADH3 (SEQ ID NO.: 27, positions 71-148) and ADH4 (SEQ ID NO.: 28, positions 72-149) are highly homologous (about 98.7%) to ADH1 (SEQ ID NO.: 1, positions 47-124), and that ADH2 (H45, H49, H122), ADH3 (H71, H75, H148) and ADH4 (H72, H76, H149) correspond to H47, H51 and H124 in ADH1 respectively, that is, the corresponding relationship among the mutation sites of ADH1, ADH2, ADH3 and ADH4 are as follows.

| ADH Family Proteins | Mutation Sites | | |
|---|---|---|---|
| ADH1 | Position 47 | Position 51 | Position 124 |
| ADH2 | Position 45 | Position 49 | Position 122 |
| ADH3 | Position 71 | Position 75 | Position 148 |
| ADH4 | Position 72 | Position 76 | Position 149 |

### WILD-TYPE ADH FAMILY PROTEIN

As used herein, "wild-type ADH family protein" refers to the naturally existing ADH family protein with no artificial engineering whose nucleotide sequence can be obtained through genetic engineering techniques (such as genome sequencing, polymerase chain reaction (PCR), etc.) and whose amino acid sequence can be deduced from the nucleotide sequence. The source of the wild-type ADH family protein is *K. lactis* wild-type cell strain. The wild-type ADH family proteins include ADH1, ADH2, ADH3 and/or ADH4 proteins.

In a preferred embodiment of the present invention, the amino acid sequence of the wild-type ADH family protein (e.g., ADH1, ADH2, ADH3, ADH4) is shown in SEQ ID NO.: 1, 26, 27 or 28, respectively.

### ADH FAMILY PROTEIN MUTANT AND CODING NUCLEIC ACID THEREOF

As used herein, the terms "mutant protein", "mutant protein of the present invention", "ADH mutant protein of the present invention", "mutated ADH protein of the present invention", "ADH mutant" and "mutant of ADH family protein" can be used interchangeably, all referring to mutant ADH protein which is not naturally existing, and the mutant protein has mutations of at least one histidine (H) among the amino acids at positions 45-149 of the wild-type alcohol dehydrogenase (ADH).

Moreover, in the present invention, the mutation does not include histidine (H) at positions 67, 69, 93 and/or 94 of the wild-type alcohol dehydrogenase (ADH).

In the present invention, the histidines can be independently mutated to basic amino acids, and the types of independent mutations of the histidine can be the same or different.

In a preferred embodiment, the histidines can be mutated to one or more amino acids selected from the following group: lysine (K), asparagine (N), glutamine (Q), arginine (R) and combinations thereof.

In a preferred embodiment, the mutant ADH protein inthe present invention is a protein obtained by artificially engineering the protein shown in SEQ ID NO: 1, 26, 27 or 28.

Wherein, the mutant protein contains core amino acids related to activity, and at least one of the core amino acids is artificially engineered; and the mutant protein in the present invention can significantly (i) improve the expression purity, efficiency, and/or yield of the exogenous protein in an *in-vitro* cell-free synthesis system; and/or (ii) reduce the binding ability of the mutant protein to Ni medium.

The term "core amino acids" refers to a sequence which is based on the sequence of SEQ ID NO.: 1, 26, 27 or 28 and has at least 80% (e.g., 84%, 85%, 90%, 92%, 95%, 98%) homology with SEQ ID NO.: 1, 26, 27 or 28, the corresponding position is the specific amino acids described herein. For example, based on the sequence shown in SEQ ID NO.: 1, the core amino acids are:
histidine (H) at position 47; and/or
histidine (H) at position 51; and/or
histidine (H) at position 124.

For example, based on the sequence shown in SEQ ID NO.: 26, the core amino acids are:
histidine (H) at position 45; and/or
histidine (H) at position 49; and/or
histidine (H) at position 122.

For example, based on the sequence shown in SEQ ID NO.: 27, the core amino acids are:
histidine (H) at position 71; and/or
histidine (H) at position 75; and/or
histidine (H) at position 148.

For example, based on the sequence shown in SEQ ID NO.: 28, the core amino acids are:
histidine (H) at position 72; and/or
histidine (H) at position 76; and/or
histidine (H) at position 149.

And the mutant proteins obtained by mutating the above-mentioned core amino acids have the activity of significantly (i) improving the expression purity, efficiency, and/or yield of the exogenous protein in an *in-vitro* cell-free synthesis system; and/or (ii) reducing the binding ability of the mutant protein to Ni medium.

In another preferred embodiment, the histidine (H) at position 47 is mutated to one or more amino acids selected from the following group: lysine (K), asparagine (N), glutamine (Q), arginine (R) and combinations thereof, preferably, lysine (K), asparagine (N) and/or arginine (R), more preferably, lysine (K) and/or asparagine (N).

In another preferred embodiment, the histidine (H) at position 51 is mutated to one or more amino acids selected from the following group: lysine (K), arginine (R) and a combination thereof, preferably, lysine (K) and/or arginine (R), more preferably, arginine (R).

In another preferred embodiment, the histidine (H) at position 124 is mutated to one or more amino acids selected from the following group: lysine (K), arginine (R) and a combination thereof, preferably, lysine (K).

In another preferred embodiment, the mutant protein has mutations at one or more core amino acids of wild-type alcohol dehydrogenase (ADH) corresponding to SEQ ID NO.: 1, wherein the one or more core amino acids selected from the following group:
histidine (H) at position 47; and
histidine (H) at position 124.

In another preferred embodiment, the histidine (H) at position 45 is mutated to one or more amino acids selected from the following group: lysine (K), arginine (R), asparagine (N), glutamine (Q) and combinations thereof.

In another preferred embodiment, histidine (H) at position 49 is mutated to one or more amino acids selected from the group consisting of lysine (K), arginine (R) and a combination thereof.

In another preferred embodiment, histidine (H) at position 122 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 26, wherein the one or more core amino acids are selected from the following group:
histidine (H) at position 122; and
histidine (H) at position 45.

In another preferred embodiment, the histidine (H) at position 71 is mutated to one or more amino acids selected from the following group: lysine (K), arginine (R), asparagine (N), glutamine (Q) and combinations thereof.

In another preferred embodiment, the histidine (H) at position 75 is mutated to one or more amino acids selected from the following group: lysine (K), arginine (R) and a combination thereof.

In another preferred embodiment, histidine (H) at position 148 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 27, wherein the one or more core amino acids are selected from the following group:
histidine (H) at position 148; and
histidine (H) at position 71.

In another preferred embodiment, the histidine (H) at position 72 is mutated to one or more amino acids selected from the following group: lysine (K), arginine (R), asparagine (N), glutamine (Q) and combinations thereof.

In another preferred embodiment, the histidine (H) at position 76 is mutated to one or more amino acids selected from the following group: lysine (K), arginine (R) and a combination thereof.

In another preferred embodiment, the histidine (H) at position 149 is mutated to one or more amino acids selected from the following group: lysine (K), arginine (R) and a combination thereof.

In another preferred embodiment, the mutant protein has mutations at one or more core amino acids of wild-type alcohol dehydrogenase (ADH) corresponding to SEQ ID NO.: 28, wherein the one or more core amino acids are selected from the following group:
histidine (H) at position 149; and
histidine (H) at position 72.

It should be understood that the amino acid serial numbers in the mutated ADH protein in the present invention are based on the amino acid sequence of the wild-type ADH protein (preferably, SEQ ID NO.: 1, 26, 27 or 28). When a specific mutant protein has at least 80% homology with the sequence shown in SEQ ID NO.: 1, 26, 27 or 28, the amino acid serial numbers of the mutant protein may have a dislocation relative to amino acid serial numbers of SEQ ID NO.: 1. 26, 27, or 28, such as 1-5 dislocation positions towards the N or C terminus of amino acids. Using conventional sequence alignment techniques in the art, it is generally understood by those skilled in the art that such dislocation is within a reasonable range, and mutant proteins having 80% (e.g. 90%, 95%, 98%) homology and having identical or similar activity, should not be excluded due to dislocation of amino acid serial numbers, from the scope of the mutant proteins in the present invention.

The mutant protein in the system of the present invention described herein is a synthesized protein or a recombinant protein, that is, it can be a chemically synthesized product, or be produced from a prokaryotic or eukaryotic host (e.g., bacteria, yeast, plant) using recombinant technique. According to the host used in the recombinant production scheme, the mutant protein can be glycosylated or non-glycosylated. The mutant protein can also include or exclude the starting methionine residue.

The system of the present invention also includes fragments, derivatives and analogues of the mutant protein. As used herein, the terms "fragment", "derivative" and "analogue" refer to a protein that substantially retains the same biological function or activity as the mutant protein.

The fragments, derivatives or analogues of the mutant protein can be (i) a mutant protein in which one or more conservative or non-conservative amino acid residues (preferably conservative amino acid residues) are substituted, whereas such resultant substituted amino acid residues may or may not be encoded by genetic codes, or (ii) a mutant protein with a substitution group in one or more amino acid residues, or (iii) a mutant protein formed by the fusion of a mature mutant protein with another compound (such as a compound that prolongs the half-life of the mutant protein, such as polyethylene glycol), or (iv) a mutant protein formed by the fusion of an additional amino acid sequence to the mutant protein sequence (such as a fusion protein formed by fusion of a leading sequence, or a secretory sequence, or a sequence used to purify this mutant protein, or a proteogen sequence, or an antigen IgG fragment with the mutant protein). According to the teachings herein, these fragments, derivatives and analogues are within the scope well known to those skilled in the art. In the present invention, conservatively substituted amino acids are preferably generated by amino acid substitutions according to Table I.

**Table I**

| Initial Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The active mutant protein has the activity of significantly (i) improving the expression purity, efficiency, and/or yield of the exogenous protein in an *in-vitro* cell-free synthesis system; and/or (ii) reducing the binding ability of the mutant protein to Ni medium.

In the system of the present invention, the mutant protein is as described in the first aspect. Preferably, the mutant protein is an amino acid sequence as shown in any one of SEQ ID NO.: 2-13.

It should be understood that the mutant protein generally has higher homology (identity) compared with the sequence shown in any one of SEQ ID NO.: 2-13. Preferably, the mutant protein has at least 80%, preferably at least 85%-90%, more preferably at least 95%, and most preferably at least 98% homology with any of the sequences shown in SEQ ID NO.2-13
In addition, the mutant protein can also be modified. Modification (usually not change the primary structure) forms include: *in vivo* or *in vitro* chemically derived forms of mutant protein, such as acetylation or carboxylation. Modification also includes glycosylation, such as glycosylation modification carried out in the synthesis and processing or in further processing steps of the mutant proteins. This modification can be accomplished by exposing the mutant proteins to enzymes (such as mammalian glycosylases or deglycosylases) that perform glycosylation. Modification forms further include sequences with phosphorylated amino acid residues (such as phosphotyrosine, phosphoserine, phosphothreonine). Modification forms further include mutant proteins that are modified and thereby obtain increased resistance to proteolysis or improved solubility.

The term "polynucleotide encoding the mutant protein" can be a polynucleotide encoding the mutant protein, and also can be a polynucleotide that further contains additional coding and/or non-coding sequences.

The polynucleotide in the system of the present invention can be in a form of DNA or RNA. In another preferred embodiment, the nucleotide is DNA. DNA forms include cDNA, genomic DNA and synthetic DNA. DNA can be single-stranded or double-stranded. DNA can be a coding strand or a non-coding strand. The coding region sequence encoding a mature polypeptide can be the same as the sequence encoding the polypeptide shown in any one of SEQ ID NO.: 2-13 or can be a degenerate variant. As used herein, "degenerate variant" in the present invention refers to a nucleic acid sequence that encodes a polypeptide shown in any one of SEQ ID NO.: 2-13 but differs in the sequence of the corresponding coding region.

The present invention also relates to variants of the aforementioned polynucleotides. The variants encode fragments, analogues and derivatives of polypeptides or mutant proteins having the same amino acid sequence as that of the present invention. These nucleotide variants include substitution variants, deletion variants and insertion variants. As is known in the art, an allelic variant is an alternative form of a polynucleotide. The allelic variant may be a substitution, deletion or insertion of one or more nucleotides, but it does not substantially change the function of the mutant protein encoded by it.

The nucleic acid sequence can be DNA, RNA, cDNA or PNA. The nucleic acid sequence can be genomic, recombinant or synthetic. The nucleic acid sequence can be isolated or purified. The nucleic acid sequence can be single-stranded or double-stranded. Preferably, the nucleic acid sequence will encode a photosensitive protein as described herein. The nucleic acid sequence can be derived by cloning techniques, such as, by standard molecular cloning techniques including restriction enzyme digestion, ligation and gel electrophoresis, such as cloning techniques described in Sambrook et al. Molecular Cloning: A laboratory manual, Cold Spring Harbour Laboratory Press. The nucleic acid sequence can be isolated, for example be isolated by using PCR technology. Isolation means the isolation of nucleic acid sequences from any impurities and from other nucleic acid sequences and/or proteins that are naturally found associated with the nucleic acid sequence in the source. Preferably, the nucleic acid sequence also does not contain cellular materials, media or other chemicals from purification/production processes. The nucleic acid sequence can be synthetic, for example can be produced by direct chemical synthesis. The nucleic acid sequence can be provided as a naked nucleic acid, or can be provided in a form of complex with proteins or lipids.

The system of the present invention also relates to a polynucleotide that hybridizes with the aforementioned sequence and has at least 50%, preferably at least 70%, and more preferably at least 80% identity with the aforementioned sequence. The system of the present invention particularly relates to a polynucleotide that can hybridize with the polynucleotide under strict conditions (or rigorous conditions). In the present invention, "strict conditions" refer to: (1) hybridization and elution at lower ionic strength and higher temperature, such as 0.2×SSC, 0.1% SDS, 60 °C; or (2) hybridization in the presence of denaturant such as 50% (v/v) formamide, 0.1% calf serum/0.1% Ficoll, 42 °C, etc.; or (3) hybridization occurs only when the identity between the two sequences is at least 90%, preferably at least 95%.

Mutant proteins and polynucleotides in the system of the present invention are preferably provided in an isolated form, more preferably, being purified to homogeneity.

The full-length polynucleotide sequence can usually be obtained by PCR amplification method, recombination method or artificial synthesis method. For the PCR amplification method, primers can be designed according to the relevant nucleotide sequence disclosed in the present invention, especially the open reading frame sequence, and the relevant sequences can be amplified using a commercially available cDNA library or a cDNA library prepared according to the conventional methods known to those skilled in the art as a template. When the sequence is long, it is often necessary to perform PCR amplifications twice or more times, and then the amplified fragments obtained from each of the PCR amplifications are spliced together in correct order.

Once related sequences are obtained, related sequences can be obtained in large numbers by recombination method. This usually refers to cloning the related sequences into vectors, transformation into cells, and subsequent isolation from the proliferated host cells by conventional methods to obtain the related sequences.

In addition, the related sequences, especially related sequences with short fragment lengths, can also be synthesized by artificial synthesis methods. Usually, synthesize many small fragments first and then carry out ligation to obtain fragments with a long sequence.

At present, the DNA sequence encoding the protein (or fragment or derivative thereof) of the system of the present invention has can be obtained entirely via chemical synthesis. The DNA sequence can then be introduced into various existing DNA molecules (such as vectors) and cells known in the art. In addition, one can also introduce mutations into the protein sequence via chemical synthesis.

The methods of amplifying DNA/RNA using PCR technology are preferably used to obtain the polynucleotide of the system of the present invention. Especially when it is difficult to obtain full-length cDNA from the library, RACE method (RACE, Rapid Amplification of cDNA ends) can be preferably used. Primers used for PCR can be appropriately selected according to the sequence information disclosed herein and can be synthesized by conventional methods. Amplified DNA/RNA fragments can be separated and purified by conventional methods such as gel electrophoresis.

In the system of the present invention described herein, DNA coding sequence of the ADH protein mutant is the nucleotide sequence shown in any one of SEQ ID NO.: 14-25.

### EXPRESSION VECTOR AND HOST CELL

Below are described a vector containing the polynucleotide of the system of the present invention, a host cell produced by genetic engineering using the vector or using the coding sequence of the mutant protein of the system of the present invention, and a method for producing the polypeptide through recombinant technology.

Through conventional recombinant DNA technology, the polynucleotide sequence can be used to express or produce recombinant mutant protein. Generally speaking, the method comprises the following steps:
(1) transforming or transducing an appropriate host cell with a polynucleotide (or a variant) encoding the mutant protein of the system of the present invention, or with a recombinant expression vector containing the polynucleotide;
(2) culturing the host cell in an appropriate medium; and
(3) isolating and purifying proteins from the medium or cells.

In the present invention, the polynucleotide sequence encoding the mutant protein can be inserted into a recombinant expression vector. The term "recombinant expression vector" refers to a bacterial plasmid, bacteriophage, yeast plasmid, plant cell virus, mammalian cell virus such as adenovirus and retrovirus, or other vectors well known in the art. Any plasmids and vectors can be used as long as they can be replicated and stable in the host. An important feature of the expression vector is that it usually contains a replication origin, a promoter, a marker gene and translation control elements.

Methods well known to those skilled in the art can be used to construct an expression vector containing the DNA sequence encoding the mutant protein of the system of the present invention and appropriate transcription/translation control signals. These methods include *in-vitro* recombinant DNA technology, DNA synthesis technology, *in-vivo* recombination technology, etc. The DNA sequence can be effectively linked to an appropriate promoter in the expression vector to guide mRNA synthesis. Representative examples of these promoters include: lac or trp promoter of *E. coli;* PL promoter of λ phage; eukaryotic promoters including CMV immediate-early promoter, HSV thymidine kinase promoter, SV40 early and late promoters, LTRs of retroviruses and some other known promoters that can control gene expression in prokaryotic or eukaryotic cells or viruses therein. The expression vector also includes a ribosome binding site for translation initiation and a transcription terminator.

In addition, the expression vector preferably contains one or more selective marker genes to provide phenotypic characters for selecting transformed host cells, such as dihydrofolate reductase, neomycin resistance and green fluorescent protein (GFP) for eukaryotic cell culture, or such as tetracycline resistance or ampicillin resistance for *E. coli.*

A vector containing the above-mentioned appropriate DNA sequence and an appropriate promoter or control sequence can be used to transform an appropriate host cell to enable it express proteins.

The host cells can be prokaryotic cells (such as *E. coli*), or lower eukaryotic cells, or higher eukaryotic cells such as yeast cells, plant cells, or mammal cells (including human and non-human mammal cells). Representative examples include: *E*. *coli,* wheat germ cells, insect cells, SF9 cells, Hela cells, HEK293 cells, CHO cells, yeast cells, etc. In a preferred embodiment of the present invention, yeast cells (such as *Pichia pastoris, Kluyveromyces* or a combination thereof; preferably, the yeast cells include: *Kluyveromyces,* more preferably *Kluyveromyces marxianus* and/or *Kluyveromyces lactis*) are selected as host cells.

When the polynucleotide of the system of the present invention is expressed in higher eukaryotic cells, transcription will be enhanced if an enhancer sequence is inserted into the vector. The enhancer is a cis-acting element of DNA, usually of about 10 to 300 base pairs, acting on promoters to enhance gene transcription. Examples include SV40 enhancer of 100 to 270 base pairs on the late side of replication origin, polyoma enhancer on the late side of replication origin, adenovirus enhancer, etc.

Those of ordinary skill in the art know how to select appropriate vectors, promoters, enhancers and host cells.

Transformation of host cells by using recombinant DNA can be performed by using conventional techniques well known to those skilled in the art. When the host is a prokaryotic organism such as *E. coli,* competent cells that can absorb DNA can be harvested after the exponential growth phase and be treated by CaCl₂ method, where the steps used are well known in the art. Another method is using MgCl₂. If necessary, transformation can also be performed by electroporation. When the host is a eukaryote, the following DNA transfection methods can be selected: calcium phosphate coprecipitation method and conventional mechanical methods such as microinjection, electroporation, liposomal packaging, etc.

The obtained transformants can be cultured by conventional methods to express the polypeptide encoded by the gene of the system of the present invention described herein. According to the host cell used, the medium used in the culture can be selected from various conventional media. The culture is carried out under a condition suitable for the growth of the host cell. After the host cell has grown to an appropriate cell density, the selected promoters are induced by appropriate methods (such as temperature switching or chemical induction) and the cells are cultured for another period of time.

The recombinant polypeptide mentioned in the above method can be expressed in the inside of the cell or on the cell membrane, or be secreted to the outside of the cell. If necessary, the recombinant protein can be separated and purified via various separation methods by virtue of its physical, chemical and other characteristics. These methods are well known to those skilled in the art. Examples of these methods include, but are not limited to: conventional renaturation treatment, treatment with protein-precipitating agent (salting-out method), centrifugation, osmotic lysis of bacteria, ultra-treatment, ultracentrifugation, molecular sieve chromatography (gel filtration), adsorption chromatography, ion exchange chromatography, high performance liquid chromatography (HPLC) and other various liquid chromatography techniques and combinations of these methods.

**The main advantages of the present invention include as follows:**
(1) The present invention determines the type of intracellular protein that can bind to Ni medium via mass spectrometry identification of the components in cells that bind to Ni medium;
(2) For the first time, the present invention provides a series of histidine sites in ADH family proteins that may bind to Ni medium by systematically analyzing ADH family proteins in the cell genome.
(3) For the first time, the present invention obtains several strains that can significantly reduce the binding ability of ADH1 to Ni medium through gene knockout of ADH1 and site-directed mutation of a series of histidine sites in ADH1 that may bind to Ni medium.
(4) By analyzing the transcription and translation activity of cell-free system of multiple cell strains mentioned above, it is found that the site-directed mutation of ADH1 has less effect on the transcription and translation activity of cell-free system than the gene knockout, and for the first time, a cell strain, which does not affect the transcription and translation activity of cell-free system as well as significantly reduces the binding ability of ADH1 to Ni medium, is obtained.
(5) The present invention takes *Kluyveromyces lactis* (*K. lactis*) as an example, but the same design, analysis, and experimental methods are also applicable to other cells including prokaryotic cells, eukaryotic cells, yeast cells, human source cells, Hela cells, CHO cells, HEK293 cells, *Saccharomyces cerevisiae,* etc.
(6) For the first time, it is found, in the present invention, that the mutation of the core amino acids of ADH family proteins can significantly (i) improve the expression purity, efficiency, and/or yield of the exogenous protein in an *in-vitro* cell-free synthesis system; and/or (ii) reduce the binding ability of the mutant protein to Ni medium.

The present invention will be further illustrated below in combination with specific examples (or embodiments). It should be understood that these examples (or embodiments) are provided solely for the purpose of illustration and should not be regarded as limitations to the scope of the present invention. With respect to the experimental methods without specifically described conditions in the following examples (or embodiments), one person can generally follow conventional conditions, such as conditions described in Sambrook et. al, Molecular Cloning: A Laboratory Manual (New York: Cold Spring Harbor Laboratory Press, 1989), or follow conditions recommended by the manufacturer. Unless otherwise stated, percentage and portions refer to percentage by weight and portions by weight.

Unless otherwise stated, materials and reagents used in the examples of the present invention are all commercially available products.

The present invention takes *Kluyveromyces lactis* (*K. lactis*) as an example, but the same design, analysis and experimental methods are also applicable to other cells including prokaryotic cells, eukaryotic cells, yeast cells, human source cells, Hela cells, CHO cells, HEK293 cells, *Saccharomyces cerevisiae* and other cells

### GENERAL METHOD

The present invention provides a design and method as follows: Ni-binding proteins are identified through Ni purification and mass spectrometry analysis, and then, on this basis, ADH family proteins in the genome are subjected to point mutations to reduce their binding ability to Ni medium, thereby improving the efficiency, yield and purity of proteins purified by Ni. The following steps are included:
(1) identification and analysis of ADH family proteins by the following method:
   A. polyacrylamide gel electrophoresis analysis of protein purified by Ni;
   B. mass spectrometry analysis of non-specific bands (35-50 kDa); and
   C. peptide comparison of mass spectrometry analysis results to identify the main non-specific band which was identified as ADH protein;
(2) analysis of potential binding sites of *K. lactis* ADH family proteins to Ni medium by the following method:
   A. sequence alignment of ADH1 protein in *K. lactis* and ADH1 protein in *Saccharomyces cerevisiae;*
   B. using ScADH as a template for homology modeling, and finding out the His-rich region sites in *Kl*ADH, such as H47, H51 and H124 in ADH1; H45, H49 and H122 in ADH2; H71, H75 and H148 and ADH3; H72, H76 and H149 in ADH4; and
   C. performing an engineerability analysis on His sites in this region, such as whether the engineering affects the catalytic activity of ADH, and finally selecting alternative amino acids, such as a site-directed mutation of ADH1 selected from the following group: H124K, H124R, H47K, H47R, H47N, H47Q, H51K, H51R, H124KH47K, H124KH47R, H124KH47N, H124KH47Q and combinations thereof; a site-directed mutation of ADH2 selected from the following group: H122K, H122R, H45K, H45R, H45N, H45Q, H49K, H49R, H122KH45K, H122KH45R, H122KH45N, H122KH45Q and combinations thereof; a site-directed mutation of ADH3 selected from the following group: H148K, H148R, H71K, H71R, H71N, H71Q, H75K, H75R, H148KH71K, H148KH71R, H148KH71N, H148KH71Q and combinations thereof; a site-directed mutation of ADH4 selected from the following group: H149K, H149R, H72K, H72R, H72N, H72Q, H76K, H76R, H149KH72K, H149KH72R, H149KH72N, H149KH72Q and combinations thereof;
(3) construction of Cas9-gRNA cloning vector, and the construction method is as follows:
   A. for the sequence of a specific gene, respectively designing a gRNA sequence that guides the splicing of the gene; and
   B. recombining the above gRNA sequence into a vector containing Cas9 to obtain a first vector in which the gRNA and Cas9 are co-expressed;
(4) construction of a donor DNA which is used for knock-out of the specific gene by the following method:
   A. downloading the nucleotide sequence of the specific gene from the gene database, and constructing a second vector by using sequences located at 800 bp upstream and downstream of the gene, respectively; and
   B. amplifying the second vector with primers M13F and M13R, and the obtained PCR product is concentrated by alcohol precipitation to obtain the donor DNA;
(5) construction of a donor DNA with a specific point mutation of histidine residue by the following method:
   A. downloading the nucleotide sequence of the specific gene from the gene database, and constructing a second vector by using the target gene and sequences located at 800 bp upstream and downstream of the target gene, respectively, making point mutations to parts of amino acid residues, and making synonymous mutations to gRNA; and
   B. amplifying the second vector with primers M13F and M13R, and the obtained PCR product is concentrated by alcohol precipitation to obtain the donor DNA;
(6) obtaining a cell strain with knockout or point mutation of the specific gene by the following method:
   A. transforming the first vector and the donor DNA simultaneously into competent cells; and
   B. screening out monoclonal cells for enlargement culture, extracting the genome, designing primers to amplify the ADH genes and homologous arms, and the amplified PCR products are verified by sequencing;
(7) testing the binding ability of the strain, which has been subjected to gene knockout or point mutant, to Ni medium by the following method:
   A. performing enlargement culture for the obtained strain having been subjected to gene knockout or point mutant, and performing cell lysis to obtain a cell lysate; and
   B. purifying the cell lysate through Ni medium, and analyzing the purified product via polyacrylamide gel electrophoresis;
(8) Cell-free *in vitro* protein synthesis system

A yeast cell lysate is prepared using the genetically engineered cell strain and added into an *in vitro* protein translation system. The reaction system is let stand at 20-30 °C for 2-12 hours, the absorbance value by using a multifunctional microplate reader (Perkin Elmer) is read, and the activity of the enhanced green fluorescent protein (eGFP) is detected.

### Example 1 Analysis and specific engineering of ADH family genes in K. lactis

### 1.1 Analysis of ADH family genes in K. lactis

When a His-tag-labeled target protein, which was expressed in *K. lactis,* was affinity-purified with Ni medium, there was an obvious non-specific band at 35-50 kDa. Mass spectrometry results showed that the non-specific protein was mainly derived from ADH family proteins in *K. lactis* (Table 1). ADH is a NAD(P)-dependent oxidoreductase, exists in almost all organisms and catalyzes the reversible oxidation of primary and secondary alcohols to aldehydes and ketones, respectively. The ADH family proteins in *K. lactis* include four types of *Kl*ADH1, *Kl*ADH2, *Kl*ADH3 and *Kl*ADH4, and all of them are encoded by chromosomal DNA. Among them, ADH1 gene sequence (SEQ ID NO.32) with a KEGG database code of *Kl*LA0F21010g is located at chromosome F; ADH2 gene sequence (SEQ ID NO.29) with a KEGG database code of *Kl*LA0F18260g is located at chromosome F; ADH3 gene sequence (SEQ ID NO. 30) with a KEGG database code of *Kl*LA0B09064g is located at chromosome B; ADH4 gene sequence (SEQ ID NO. 31) with a KEGG database code of KlLA0F13530g is located at chromosome F. Among them, ADH1 and ADH2 carry out their functions in the cytosol, while ADH3 and ADH4 carry out their functions in mitochondria, with relative molecular weights of 37.3 kDa, 37.1 kDa, 39.6 kDa and 40.2 kDa, respectively. The amino acid sequences of these four ADH proteins are highly homologous and their tertiary structures are also quite similar .

**Table 1 Peptide fragments obtained by identifying non-specific proteins purified by Ni medium by mass spectrometry and K. lactis endogenous proteins indicated by the peptide fragments**

| **Peptide Sequence** | **Target Protein in *K. lactis*** |
|---|---|
| EAIDFFSR | ADH1 |
| SNGTVVLVGLPR | ADH1&ADH2 |
| SISIVGSYVGNR | ADH1 |
| DLGGEYFIDFTK | ADH1 |
| GVIFYENGGELQYK | ADH1&ADH2 |
| LPLVGGHEGAGVVVAMG ENVK | ADH1&ADH2&ADH3 (mitochondria) |
| APIHVVGLSELPSIYEK | ADH1 |
| GGAHGVINVSVSEFAIEQS TNYVR | ADH1&ADH2 |
| AGDWVAISGAAGGLGSLA VQYAK | ADH1&ADH3 (mitochondria) |
| GVIFYENGGKlEYK | ADH3&ADH4 (mitochondria) |
| SDVFNQVVK | ADH1 |
| NIPEEVIEATK | ADH1 |
| ANEILINVK | ADH4 |

Because the crystal structure of ADH family proteins in *K. lactis* are still unknown, we used homology modeling to predict their spatial tertiary structures. The primary sequence alignment between *Kl*ADH1 in *K. lactis* and ScADH1 in *Saccharomyces cerevisiae* (PDB No. 4W6Z) shows that the two are tetramers with 84.9% sequence homology. Homology modeling using ScADH1 as template showed that each monomer of *Kl*ADH1 in *K. lactis* contains a total of six His, located at positions 47, 51, 69, 124, 243 and 321, respectively. The whole tetramer contains a total of four His-rich regions. Except that the 5th His of each monomer is relatively independent, the other five His of each monomer are close in space, wherein, the closest in space (and also in sequence) among the other five His are the three to four His in front of each monomer.

Since the four ADHs in *K. lactis* are highly homologous and five His are highly conserved, the spatial structures of *Kl*ADH2, KIADH3 and *Kl*ADH4 are also similar to the spatial structure of *Kl*ADH1, that is, the first four His of each ADH protein may cluster in space, thereby generating affinity to Ni medium.

### 1.2 Specific engineering of ADH family genes in K. lactis

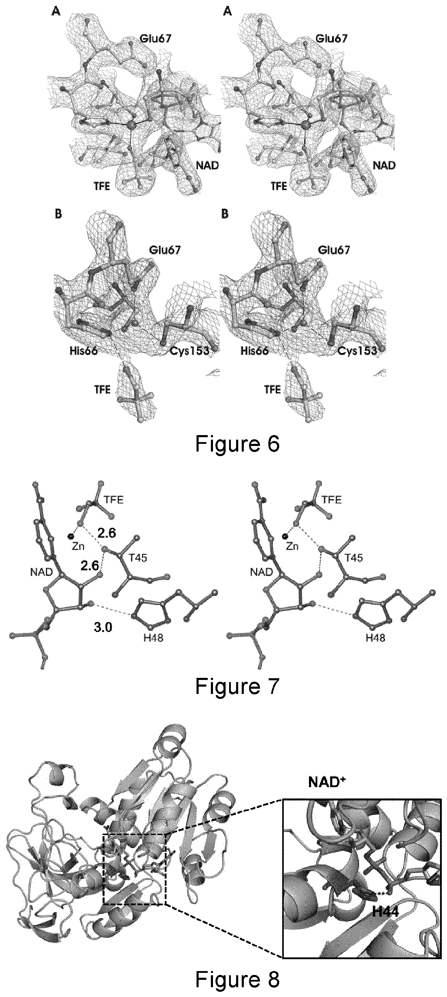

In view of the need to minimize as far as possible the effect on ADH activity when eliminating the affinity of ADH to Ni medium, the analysis of function of His in KIADH becomes the key. Through a large number of comparisons and screening, it was found that among the six His in *Kl*ADH1 protein homologous to those in ScADH1 protein, H44, H48, H66 and H121, because of their homology with the four His clustered in *Kl*ADH1, become the key point of the analysis of the present invention. Because H66 is involved in chelating catalytic Zn²⁺ in both two conformations of ScADH1, as shown in Figure 6, it may play a significant impact on the catalytic activity of ADH, so as that it is not taken as an engineering target of the present invention. Although H48 does not bind to coenzymes, it participates in the formation of the ternary complex and affects the proton transfer process of the substrate, as shown in Figure 7. The research of the present invention found that ND1 on the H44 imidazole ring may form a hydrogen bond with O2A of NAD⁺, thereby participating in the binding of NAD⁺/NADH. H44R can reduce the dissociation constant of NAD⁺ and NADH by 2-4 times, and decrease the conversion number by 4-6 times. In addition, the side chain of H121 can form a hydrogen bond with T130.

Based on the above research, in the present invention, three His sites (H44, H48 and H121, respectively) that have little effect on the function of ADH1 in ScADH1 were mutated to amino acids with similar function, and finally the point mutation scheme designed for *Kl*ADH1 was H124K, H124R, H47K, H47R, H47N, H47Q, H51K, H51R, H124KH47K, H124KH47R, H124KH47N and H124KH47Q.

Because ADH2, ADH3 and ADH4 in *K. lactis* are highly homologous with ADH1 (Figure 2), that is, H45, H49 and H122 in ADH2, H71, H75 and H148 in ADH3, and H72, H76 and H149 in ADH4 correspond to H47, H51 and H124 in ADH1, respectively.

Therefore, in the present invention, ADH1 is taken as an example to reduce the binding of ADH1 to Ni medium, specifically as indicated in the knockout of *ADH1* gene and the site-directed mutation of H47, H51 and H124. ADH2, ADH3 and ADH4 can also be engineered at corresponding sites by using methods similar to those for ADH1.

### Example 2 Targeted knockout of ADH1 gene via CRISPR/Cas9

### 2.1 Determination of KlADH1 CRISPR gRNA sequence

According to the point mutation designed in *Kl*ADH1, PAM sequence (NGG) was selected and corresponding gRNA sequence was determined. The principle for selecting gRNA in this example is as follows: the GC content should be moderate (40%-60%), and the existence of a poly T structure should be avoided. In this embodiment, the *Kl*ADH1 gRNA1 sequence is TGGGTGAAAACGTCAAGGGC (SEQ ID No.: 33).

Methods for plasmid construction and transformation are as follows: using primers pKMCas9-*Kl*ADH1-gRNA1-PF: TGGGTGAAAACGTCAAGGGCGTTTTAGAGCTAGAAATAGC (SEQ ID No.: 34) and pCas9-*Kl*ADH1-gRNA1-PR: GCCCTTGACGTTTTCACCCAAAAGTCCCATTCGCCACCCG (SEQ ID No.: 35), and using pCAS plasmid as template, PCR amplification was performed. 17 µL of amplified product was taken and added with 1 µL of Dpn I and 2 µL of 10 × digestion buffer; after mixing, the mixture was incubated at 37 °C for 3 hours. 10 µL of DpnI-treated mixture was added into 50 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Two monoclonal colonies were picked out and then cultured with shaking in an LB liquid medium. After being detected PCR-positive and confirmed by sequencing, the plasmid was extracted and stored, and named pKMCas9_StR_*KlADH1*.

### 2.2 Construction and amplification of donor DNA plasmid

In this example, to facilitate the storage and amplification of linear donor DNA, the donor DNA was firstly inserted into a pKMD1 plasmid, and then amplified by PCR to obtain linear donor DNA sequences.

A PCR amplification process was carried out by using primers of pKMD1-PF: ATCGTCGACCTGCAGGCATG (SEQ ID No.: 36) and pKMD1-PR: ATCTCTAGAGGATCCCCGGG (SEQ ID No.: 37) with a pKMD1 plasmid as template. 17 µL amplified product was taken and then added with 1 µL of Dpn I and 2 µL of 10 × digestion buffer; after mixing, the mixture was incubated at 37 °C for 3 hours; and a linear fragment pKMD1-T of plasmid skeleton was obtained.

### 2.2.1 Construction of donor plasmid pKMD1-ΔKlADH1

One PCR amplification process was carried out by using primers of KIADH1-HR1-PF: TCGAGCTCGGTACCCGGGGATCCTCTAGAGATCATGGACAATACGTTACCG AGATGAGG (SEQ ID No.: 38) and KIADH1-HR1-PR: GAAGAGATTTCATTTATCTTTTTTTAGTATAGAGTTTGTGTGTTTAAAGCTTG (SEQ ID No.: 39) with a *Kluyveromyces lactis* genomic DNA as template, and the product was named Δ*Kl*ADH1-F1; another PCR amplification process was carried out by using primers of *Kl*ADH1-HR2-PF: CAAACTCTATACTAAAAAAAGATAAATGAAATCTCTTCCGCATTCAAG TCATGAC (SEQ ID No.: 40) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named Δ*Kl*ADH1-F2.

1 µL of each of the amplified products Δ*Kl*ADH1-F1, Δ*Kl*ADH1-F2 and pKMD1-T were mixed, and added with 5 µL of Cloning Mix (a kit named Transgene pEASY-Uni Seamless Cloning and Assembly Kit, from TransGen Biotech Company, the same below) and 2 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells (from TransGen Biotech Company, the same below). The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-Δ*KlADH1*.

### 2.3 Electrotransformation of K. lactis

The competent cells were taken out from a -80 °C refrigerator, melted on ice and added with 400 ng gRNA&Cas9 plasmids (or gRNA/cas9 fragments) and 1000 ng Donor DNA fragments. After mixing, the mixture was transferred into an electroporation cuvette and was bathed in ice for 2 minutes. The electroporation cuvette was put into electroporator for electric shock (with parameters of 1.5 kV, 200 Ω and 25 µF). After completion of the electric shock, the mixture was immediately added with 700 µL of YPD, and then incubated on a shaker at a speed of 200 rpm at 30 °C for 1-3 hours. 2-200 µL of the mixture was taken and inoculated on YPD (with G418 resistance) plate and cultured at 30 °C for 2-3 days until monoclonal colonies appeared.

### 2.4 Positive identification

12 to 24 monoclonal colonies were picked out from the plate with transformed cells. Samples were detected by PCR using identification primers of ADH1-CF: GTGATGGAACACGGGAATAG (SEQ ID No.: 42) and ADH1-CR: CACATATACCTTGGCAGTAG (SEQ ID No.: 43) with the cells as template. Strains that were tested positive by PCR and identified by sequencing were determined as positive strains and named ΔADH1.

### Example 3 Site-directed mutation of ADH1 H47 site by CRISPR/Cas9

### 3.1 Determination of KlADH1 CRISPR gRNA sequence

According to the point mutation designed in *Kl*ADH1, PAM sequence (NGG) was selected and corresponding gRNA sequence was determined. The principle for selecting gRNA in this example is as follows: the gRNA should be close to the designed mutation site; the GC content should be moderate (40%-60%), and the existence of a poly T structure should be avoided. In this embodiment, the *KlADH1* gRNA1 sequence is TGGGTGAAAACGTCAAGGGC (SEQ ID No.: 33).

Methods for plasmid construction and transformation are as follows: using primers pCas9-*Kl*ADH1-gRNA1-PF: TGGGTGAAAACGTCAAGGGCGTTTTAGAGCTAGAAATAGC (SEQ ID No.: 34) and pCas9-*Kl*ADH1-gRNA1-PR: GCCCTTGACGTTTTCACCCAAAAGTCCCATTCGCCACCCG (SEQ ID No.: 35), and using pCAS plasmid as template, PCR amplification was performed. 17 µL of amplified product was taken and added with 1 µL of Dpn I and 2 µL of 10 × digestion buffer. After mixing, the mixture was incubated at 37 °C for 3 hours. 10 µL of DpnI-treated mixture was added into 50 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Two monoclonal colonies were picked out and then cultured with shaking in an LB liquid medium. After being detected PCR-positive and confirmed by sequencing, the plasmid was extracted and stored, and named pKMCas9_StR_*KlADH1*.

### 3.2 Construction and amplification of donor DNA plasmid

In this example, to facilitate the storage and amplification of linear donor DNA, the donor DNA was firstly inserted into a pKMD1 plasmid, and then amplified by PCR to obtain linear donor DNA sequences.

A PCR amplification process was carried out by using primers of pKMD1-PF: ATCGTCGACCTGCAGGCATG (SEQ ID No.: 36) and pKMD1-PR: ATCTCTAGAGGATCCCCGGG (SEQ ID No.: 37) with a pKMD1 plasmid as template. 17 µL amplified product was taken and then added with 1 µL of Dpn I and 2 µL of 10 × digestion buffer; after mixing, the mixture was incubated at 37 °C for 3 hours; and a linear fragment pKMD1-T of plasmid skeleton was obtained.

### 3.2.1 Construction of donor plasmid pKMD1-KlADH1-H47K

One PCR amplification process was carried out by using primers of KIADH1-HR1-PF: TCGAGCTCGGTACCCGGGGATCCTCTAGAGATCATGGACAATACGTTACCG AGATGAGG (SEQ ID No.: 38) and KIADH1-H47K-PR: AGCATGAAGGTCAGTTTTACAGACACCGGAGTACTTGACG (SEQ ID No.: 44) with a *Kluyveromyces lactis* genomic DNA as template, and the product was named KIADH1-H47K-F1; another PCR amplification process was carried out by using primers of KIADH1-H47K-PF: GTAAAACTGACCTTCATGCTTGGAAGGGTGACTGGCCTTTG (SEQ ID No.: 45) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named KIADH1-H47K-F2; another PCR amplification process was carried out by using primers of *Kl*ADH1-gRNA1-m-PF : GGGAGAGAATGTTAAAGGTTGGAAGATTGGTGACTTCGCTGG (SEQ ID No.: 47) and *Kl*ADH1-HR2-PR : TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47N-F3.

1 µL of each of the amplified products *Kl*ADH1-H47K-F1, KIADH1-H47K-F2, KIADH1-H47K-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H47K.

### 3.2.2 Construction of donor plasmid pKMD1-KlADH1-H47N

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and KIADH1-H47N-PR: AGCATGAAGGTCAGTATTACAGACACCGGAGTACTTGACG (SEQ ID No.: 48) with a *Kluyveromyces lactis* genomic DNA as template, and the product was named KIADH1-H47N-F1; another PCR amplification process was carried out by using primers of KIADH1-H47N-PF: GTAATACTGACCTTCATGCTTGGAAGGGTGACTGGCCTTTG (SEQ ID No.: 49) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47N-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47N-F3.

1 µL of each of the amplified products *Kl*ADH1-H47N-F1, KIADH1-H47N-F2, KIADH1-H47N-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1-*H47N.

### 3.2.3 Construction of donor plasmid pKMD1-KlADH1-H47Q

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-H47Q-PR: AGCATGAAGGTCAGTTTGACAGACACCGGAGTACTTGACG (SEQ ID No.: 50) with a *Kluyveromyces lactis* genomic DNA as template, and the product was named KIADH1-H47Q-F1; another PCR amplification process was carried out by using primers of KIADH1-H47Q-PF: GTCAAACTGACCTTCATGCTTGGAAGGGTGACTGGCCTTTG (SEQ ID No.: 51) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47Q-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47Q-F3.

1 µL of each of the amplified products *Kl*ADH1-H47Q-F1, KIADH1-H47Q-F2, KIADH1-H47Q-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H47Q.

### 3.2.4 Construction of donor plasmid pKMD1-KlADH1-H47R

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and KIADH1-H47R-PR: AGCATGAAGGTCAGTTCTACAGACACCGGAGTACTTGACG (SEQ ID No.: 52) with a *Kluyveromyces lactis* genomic DNA as template, and the product was named KIADH1-H47R-F1; another PCR amplification process was carried out by using primers of KIADH1-H47R-PF: GTAGAACTGACCTTCATGCTTGGAAGGGTGACTGGCCTTTG (SEQ ID No.: 53) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47R-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47R-F3.

1 µL of each of the amplified products *Kl*ADH1-H47R-F1, *Kl*ADH1-H47R-F2, K*l*ADH1-H47R-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H47R.

### 3.3 Electrotransformation of K. lactis (the same as Example 2.3)

### 3.4 Positive identification

12 to 24 monoclonal colonies were picked out from a plate with transformed cells. Samples were detected by PCR using identification primers of ADH1-m-CF: TGGGAGAGAATGTTAAAGGT (SEQ ID No.: 54) and ADH1-m-CR: TGACGGTCGTTAACTAAGAT (SEQ ID No.: 55) and by using the cells as template. Strains that were tested positive by PCR and identified by sequencing were determined as positive strains and named ADH1 H47K, ADH1 H47N, ADH1 H47Q and ADH1 H47R, respectively.

### Example 4 Site-directed mutation of ADH1 H51 site by CRISPR/Cas9

### 4.1 Determination of KlADH1 CRISPR gRNA sequence

According to the point mutation designed in *Kl*ADH1, PAM sequence (NGG) was selected and corresponding gRNA sequence was determined. The principle for selecting gRNA in this example is as follows: the gRNA should be close to the designed mutation site; the GC content should be moderate (40%-60%), and the existence of a poly T structure should be avoided. In this embodiment, the *KlADH1* gRNA1 sequence is TGGGTGAAAACGTCAAGGGC (SEQ ID No.: 33).

Methods for plasmid construction and transformations are as follows: using primers pCas9-*Kl*ADH1-gRNA1-PF: TGGGTGAAAACGTCAAGGGCGTTTTAGAGCTAGAAATAGC (SEQ ID No.: 34) and pCas9-*Kl*ADH1-gRNA1-PR: GCCCTTGACGTTTTCACCCAAAAGTCCCATTCGCCACCCG (SEQ ID No.: 35), and using pCAS plasmid as template, PCR amplification was performed. 17 µL of amplified product was taken and added with 1 µL of Dpn I and 2 µL of 10 × digestion buffer. After mixing the mixture was incubated at 37 °C for 3 hours. 10 µL of DpnI-treated mixture was added into 50 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Two monoclonal colonies were picked out and then cultured with shaking in an LB liquid medium. After being detected PCR-positive and confirmed by sequencing, the plasmid was extracted and stored, and named pKMCas9_StR_*KlADH1*.

### 4.2 Construction and amplification of donor DNA plasmid

In this example, to facilitate the storage and amplification of linear donor DNA, the donor DNA was firstly inserted into a pKMD1 plasmid, and then amplified by PCR to obtain linear donor DNA sequences.

One PCR amplification process was carried out by using primers of pKMD1-PF: ATCGTCGACCTGCAGGCATG (SEQ ID No.: 36) and pKMD1-PR: ATCTCTAGAGGATCCCCGGG (SEQ ID No.: 37) with a pKMD1 plasmid as template. 17 µL amplified product was taken and then added with 1 µL of Dpn I and 2 µL of 10 × digestion buffer; after mixing, the mixture was incubated at 37 °C for 3 hours; and a linear fragment pKMD1-T of plasmid skeleton was obtained.

### 4.2.1 Construction of donor plasmid pKMD1-KlADH1-H51K

One PCR amplification process was carried out by using primers of KIADH1-HR1-PF: TCGAGCTCGGTACCCGGGGATCCTCTAGAGATCATGGACAATACGTTACCG AGATGAGG (SEQ ID No.: 38) and KIADH1-H51K-PR: CCAAGCTTTAAGGTCAGTATGACAGACACCGGAGTACTTGACG (SEQ ID No.: 56) with a *Kluyveromyces lactis* genomic DNA as template, and the product was named *Kl*ADH1-H51K-F1; another PCR amplification process was carried out by using primers of *Kl*ADHI1-H51K-PF: GTCATACTGACCTTAAAGCTTGGAAGGGTGACTGGCCTTTG (SEQ ID No.: 57) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H51K-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H51K-F3.

1 µL of each of the amplified products *Kl*ADH1-H51K-F1, KIADH1-H51K-F2, *Kl*ADH1-H51K-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H51K.

### 4.2.2 Construction of donor plasmid pKMD1-KIADHI-H51R

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and KIADH1-H51R-PR: CCAAGCTCTAAGGTCAGTATGACAGACACCGGAGTACTTGACG (SEQ ID No.: 58) with a *Kluyveromyces lactis* genomic DNA as template, and the product was named *Kl*ADH1-H51R-F1; another PCR amplification process was carried out by using primers of *Kl*ADH1-H51R-PF: GTCATACTGACCTTAGAGCTTGGAAGGGTGACTGGCCTTTG (SEQ ID No.: 59) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H51R-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and KIADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H51R-F3.

1 µL of each of the amplified products KIADH1-H51R-F1, KIADH1-H51R-F2, KIADH1-H51R-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H51R.

### 4.3 Electrotransformation of K. lactis (the same as Example 2.3)

### 4.4 Positive identification

12 to 24 monoclonal colonies were picked out from a plate with transformed cells. Samples were detected by PCR using identification primers of ADH1-m-CF: TGGGAGAGAATGTTAAAGGT (SEQ ID No.: 54) and ADH1-m-CR: TGACGGTCGTTAACTAAGAT (SEQ ID No.: 55) with the cells as template. Strains that were tested positive by PCR and identified by sequencing were determined as positive strains and named ADH1 H51K and ADH1 H51R, respectively.

### Example 5 Site-directed mutation of ADH1 H124 site by CRISPR/Cas9

### 5.1 Determination of KlADH1 CRISPR gRNA sequence

According to the point mutation designed in *Kl*ADH1, PAM sequence (NGG) was selected and corresponding gRNA sequence was determined. The principle for selecting gRNA in this example is as follows: the gRNA should be close to the designed mutation site; the GC content should be moderate (40%-60%), and the existence of a poly T structure should be avoided. In this embodiment, the *KlADH1* gRNA1 sequence is TGGGTGAAAACGTCAAGGGC (SEQ ID No.: 33).

Methods for plasmid construction and transformation of are as follows: using primers pCas9-*Kl*ADH1-gRNA1-PF: TGGGTGAAAACGTCAAGGGCGTTTTAGAGCTAGAAATAGC (SEQ ID No.: 34) and pCas9-*Kl*ADH1-gRNA1-PR: GCCCTTGACGTTTTCACCCAAAAGTCCCATTCGCCACCCG (SEQ ID No.: 35), and using pCAS plasmid as template, PCR amplification was performed. 17 µL of amplified product was taken and added with 1 µL of Dpn I and 2 µL of 10 × digestion buffer; after mixing, the mixture was incubated at 37 °C for 3 hours. 10 µL of DpnI-treated mixture was added into 50 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Two monoclonal colonies were picked out and then cultured with shaking in an LB liquid medium. After being detected PCR-positive and confirmed by sequencing, the plasmid was extracted and stored, and named pKMCas9_StR_*KlADH1*.

### 5.2 Construction and amplification of donor DNA plasmid

In this example, to facilitate the storage and amplification of linear donor DNA, the donor DNA was firstly inserted into a pKMD1 plasmid, and then amplified by PCR to obtain linear donor DNA sequences.

A PCR amplification process was carried out by using primers of pKMD1-PF: ATCGTCGACCTGCAGGCATG (SEQ ID No.: 36) and pKMD1-PR: ATCTCTAGAGGATCCCCGGG (SEQ ID No.: 37) with a pKMD1 plasmid as template. 17 µL amplified product was taken and then added with 1 µL of Dpn I and 2 µL of 10 × digestion buffer; after mixing, the mixture was incubated at 37 °C for 3 hours; and a linear fragment pKMD1-T of plasmid skeleton was obtained.

### 5.2.1 Construction of donor plasmid pKMD1-KlADHI-H124K

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H124K-F1; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124K-PR: CCATCTTTTGTATATCCACTCAAGTCAGCTTCTGGACAGTTGG (SEQ ID No.: 60) with a *Kluyveromyces lactis* genomic DNA as template, and the product was named *Kl*ADH1-H124K-F2; another PCR amplification process was carried out by using primers of *Kl*ADH1-H124K-PF: CTTGAGTGGATATACAAAAGATGGTTCTTTCCAACAATACGCTACTGC (SEQ ID No.: 61) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H124K-F3.

1 µL of each of the amplified products *Kl*ADH1-H124K-F1, *Kl*ADH1-H124K-F2, *Kl*ADH1-H124K-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H124K.

### 5.2.2 Construction of donor plasmid pKMD1-KIADHI-H124R

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H124K-F1; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124R-PR: CCATCTCTTGTATATCCACTCAAGTCAGCTTCTGGACAGTTGG (SEQ ID No.: 62) with a *Kluyveromyces lactis* genomic DNA as template, and the product was named *Kl*ADH1-H124R-F2; another PCR amplification process was carried out by using primers of *Kl*ADH1-H124R-PF: CTTGAGTGGATATACAAGAGATGGTTCTTTCCAACAATACGCTACTGC (SEQ ID No.: 63) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H124R-F3.

1 µL of each of the amplified products *Kl*ADH1-H124R-F1, *Kl*ADH1-H124R-F2, *Kl*ADH1-H124R-F3 and pKMD1-T were mixed, and added with 5 µL of Cloning Mix and 1 µL of water. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H124R.

### 5.3 Electrotransformation of K. lactis (the same as Example 2.3)

### 5.4 Positive identification

12 to 24 monoclonal colonies were picked out from a plate with transformed cells. Samples were detected by PCR using identification primers of ADH1-m-CF: TGGGAGAGAATGTTAAAGGT (SEQ ID No.: 54) and ADH1-m-CR: TGACGGTCGTTAACTAAGAT (SEQ ID No.: 55) with the cells as template. Strains that were tested positive by PCR and identified by sequencing were determined as positive strains and named ADH1 H124K and ADH1 H124R, respectively.

### Example 6 Simultaneous site-directed mutation of ADH1 H47 and H124 sites by CRISPR/Cas9

### 6.1 Determination of KlADH1 CRISPR gRNA sequence

According to the point mutation designed in KlADH1, PAM sequence (NGG) was selected and corresponding gRNA sequence was determined. The principle for selecting gRNA in this example is as follows: the gRNA should be close to the designed mutation site; the GC content should be moderate (40%-60%), and the existence of a poly T structure should be avoided. In this embodiment, the *Kl*ADH1 gRNA1 sequence is TGGGTGAAAACGTCAAGGGC (SEQ ID No.: 33).

Methods for plasmid construction and transformation of are as follows: using primers pCas9-*Kl*ADH1-gRNA1-PF: TGGGTGAAAACGTCAAGGGCGTTTTAGAGCTAGAAATAGC (SEQ ID No.: 34) and pCas9-*Kl*ADH1-gRNA1-PR: GCCCTTGACGTTTTCACCCAAAAGTCCCATTCGCCACCCG (SEQ ID No.: 35), and using pCAS plasmid as template, PCR amplification was performed. 17 µL of amplified product was taken and added with 1 µL of Dpn I and 2 µL of 10 × digestion buffer; after mixing, the mixture was incubated at 37 °C for 3 hours. 10 µL of DpnI-treated mixture was added into 50 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Two monoclonal colonies were picked out and then cultured with shaking in an LB liquid medium. After being detected PCR-positive and confirmed by sequencing, the plasmid was extracted and stored, and named pKMCas9_StR_*KlADH1.*

### 6.2 Construction and amplification of donor DNA plasmid

In this example, to facilitate the storage and amplification of linear donor DNA, the donor DNA was firstly inserted into a pKMD1 plasmid, and then amplified by PCR to obtain linear donor DNA sequences.

A PCR amplification process was carried out by using primers of pKMD1-PF: ATCGTCGACCTGCAGGCATG (SEQ ID No.: 36) and pKMD1-PR: ATCTCTAGAGGATCCCCGGG (SEQ ID No.: 37) with a pKMD1 plasmid as template. 17 µL amplified product was taken and then added with 1 µL of Dpn I and 2 µL of 10 × digestion buffer; after mixing, the mixture was incubated at 37 °C for 3 hours; and a linear fragment pKMD1-T of plasmid skeleton was obtained.

### 6.2.1 Construction of donor plasmid pKMD1-KlADH1-H47KH124K

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-H47K-PR: AGCATGAAGGTCAGTTTTACAGACACCGGAGTACTTGACG (SEQ ID No.: 44) with a *Kluyveromyces lactis* genomic DNA as template, and the product was named *Kl*ADH1-H47KH124K-F1; another PCR amplification process was carried out by using primers of *Kl*ADH1-H47K-PF: GTAAAACTGACCTTCATGCTTGGAAGGGTGACTGGCCTTTG (SEQ ID No.: 45) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47KH124K-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124K-PR: CCATCTTTTGTATATCCACTCAAGTCAGCTTCTGGACAGTTGG (SEQ ID No.: 60) with a *Kluyveromyces lactis* genomic DNA as template, and the product was named *Kl*ADH1-H47KH124K-F3; another PCR amplification process was carried out by using primers of *Kl*ADH1-H124K-PF: CTTGAGTGGATATACAAAAGATGGTTCTTTCCAACAATACGCTACTGC (SEQ ID No.: 61) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47KH124K-F4.

1 µL of each of the amplified products *Kl*ADH1-H47KH124K-F1, *Kl*ADH1-H47KH124K-F2, *Kl*ADH1-H47KH124K-F3, *Kl*ADH1-H47KH124K-F4 and pKMD1-T were mixed, and added with 5 µL of Cloning Mix. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*Kl*ADH1-H47KH124K.

### 6.2.2 Construction of donor plasmid pKMD1-KlADH1-H47NH124K

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-H47N-PR: AGCATGAAGGTCAGTATTACAGACACCGGAGTACTTGACG (SEQ ID No.: 48) with a *Kluyveromyces lactis* genomic DNA as template, and the product was named *Kl*ADH1-H47NH124K-F1; another PCR amplification process was carried out by using primers of *Kl*ADH1-H47N-PF: GTAATACTGACCTTCATGCTTGGAAGGGTGACTGGCCTTTG (SEQ ID No.: 49) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47NH124K-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124K-PR: CCATCTTTTGTATATCCACTCAAGTCAGCTTCTGGACAGTTGG (SEQ ID No.: 60) with a *Kluyveromyces lactis* genomic DNA as template, and the product was named *Kl*ADH1-H47NH124K-F3; another PCR amplification process was carried out by using primers of *Kl*ADH1-H124K-PF: CTTGAGTGGATATACAAAAGATGGTTCTTTCCAACAATACGCTACTGC (SEQ ID No.: 61) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47NH124K-F4.

1 µL of each of the amplified products *Kl*ADH1-H47NH124K-F1, *Kl*ADH1-H47NH124K-F2, *Kl*ADH1-H47NH124K-F3, *Kl*ADH1-H47NH124K-F4 and pKMD1-T were mixed, and added with 5 µL of Cloning Mix. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*Kl*ADH1-H47NH124K.

### 6.2.3 Construction of donor plasmid pKMD1-KlADH1-H47QH124K

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-H47Q-PR: AGCATGAAGGTCAGTTTGACAGACACCGGAGTACTTGACG (SEQ ID No.: 50) with a *Kluyveromyces lactis* genomic DNA as template, and the product was named *Kl*ADH1-H47QH124K-F1; another PCR amplification process was carried out by using primers of *Kl*ADH1-H47Q-PF: GTCAAACTGACCTTCATGCTTGGAAGGGTGACTGGCCTTTG (SEQ ID No.: 51) and *Kl*ADH1-gRNA1-m-PR: CCAACCTTTAACATTCTCTCCCATAGCAACAACGACACCAG (SEQ ID No.: 46) with a *Kluyveromyces lactis* genomic DNA as template, and the product was named *Kl*ADH1-H47QH124K-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124K-PR: CCATCTTTTGTATATCCACTCAAGTCAGCTTCTGGACAGTTGG (SEQ ID No.: 60) with a *Kluyveromyces lactis* genomic DNA as template, and the product was named *Kl*ADH1-H47QH124K-F3; another PCR amplification process was carried out by using primers of *Kl*ADH1-H124K-PF: CTTGAGTGGATATACAAAAGATGGTTCTTTCCAACAATACGCTACTGC (SEQ ID No.: 61) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47QH124K-F4.

1 µL of each of the amplified products *K*lADH1-H47QH124K-F1, *Kl*ADH1-H47QH124K-F2, *Kl*ADH1-H47QH124K-F3, *Kl*ADH1-H47QH124K-F4 and pKMD1-T were mixed, and added with 5 µL of Cloning Mix. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H47QH124K.

### 6.2.4 Construction of donor plasmid pKMD1-KlADH1-H47RH124K

One PCR amplification process was carried out by using primers of *Kl*ADH1-HR1-PF: TCGAGCTCGGTACCCGGGGATCCTCTAGAGATCATGGACAATACGTTACCG AGATGAGG (SEQ ID No.: 38) and *Kl*ADH1-H47R-PR: AGCATGAAGGTCAGTTCTACAGACACCGGAGTACTTGACG (SEQ ID No.: 52) with a *Kluyveromyces lactis* genomic DNA as template, and the product was named *Kl*ADH1-H47RH124K-F1; another PCR amplification process was carried out by using primers of *Kl*ADH1-H47R-PF: GTAGAACTGACCTTCATGCTTGGAAGGGTGACTGGCCTTTG (SEQ ID No.: 53) and *Kl*ADH1-gRNA1-m-PR : CCAACCTTTAACATTCTCTCCCATAGCAACAACGACACCAG (SEQ ID No.: 46) with a *Kluyveromyces lactis* genomic DNA as template, and the product was named *Kl*ADH1-H47RH124K-F2; another PCR amplification process was carried out by using primers of *Kl*ADH1-gRNA1-m-PF: GGGAGAGAATGTTAAAGGTTGGAAGATTGGTGACTTCGCTGG (SEQ ID No.: 47) and *Kl*ADH1-H124K-PR: CCATCTTTTGTATATCCACTCAAGTCAGCTTCTGGACAGTTGG (SEQ ID No.: 60) with a *Kluyveromyces lactis* genomic DNA as template, and the product was named *Kl*ADH1-H47RH124K-F3; another PCR amplification process was carried out by using primers of *Kl*ADH1-H124K-PF: CTTGAGTGGATATACAAAAGATGGTTCTTTCCAACAATACGCTACTGC (SEQ ID No.: 61) and *Kl*ADH1-HR2-PR: TGCCAAGCTTGCATGCCTGCAGGTCGACGATCTTATACTGGGTAGATTTCAA ACGGGAC (SEQ ID No.: 41) with a *Kluyveromyces lactis* genomic DNA as template, and the product was named *Kl*ADH1-H47RH124K-F4.

1 µL of each of the amplified products *Kl*ADH1-H47RH124K-F1, *Kl*ADH1-H47RH124K-F2, *Kl*ADH1-H47RH124K-F3, *Kl*ADH1-H47RH124K-F4 and pKMD1-T were mixed, and added with 5 µL of Cloning Mix. After mixing, the mixture was bathed in 50 °C water for 1 hour. After the water bath, the mixture was placed on ice for 2 minutes. 10 µL of the reaction mixture was all added into 50 µL of Trans-T1 competent cells. The mixture was placed on ice for 30 minutes, heat-shocked at 42 °C for 30 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Six monoclonal colonies were picked out and then cultured with shaking in an LB liquid medium. After being detected PCR-positive and confirmed by sequencing, the plasmid was extracted and stored, and named pKMD1-*KlADH1*-H47RH124K.

### 6.3 Electrotransformation of K. lactis (the same as Example 2.3)

### 6.4 Positive identification

12 to 24 monoclonal colonies were picked out from a plate with transformed cells. Samples were detected by PCR using identification primers of ADH1-m-CF: TGGGAGAGAATGTTAAAGGT (SEQ ID No.: 54) and ADH1-m-CR: TGACGGTCGTTAACTAAGAT (SEQ ID No.: 55) with the cells as template. Strains that were tested positive by PCR and identified by sequencing were determined as positive strains and named ADH1 H47KH124K, ADH1 H47NH124K, ADH1 H47QH124K and ADH1 H47RH124K, respectively.

### Example 7 Analysis of binding ability of endogenous protein of KlADH1-engineered cell strains to Ni medium

### 7.1 Reagent preparation for protein purification system

Binding buffer: 0.5 M NaCl, 20 mM Tris-HCl, 5 mM imidazole, pH 7.9.
Wash buffer: 0.5 M NaCl, 20 mM Tris-HCl, 60 mM imidazole, pH 7.9.

### 7.2 Purification of endogenous protein in KlADH1-engineered cell strains and analysis of their binding ability to Ni medium

Ni-NTA beads were balanced by binding buffer; an appropriate amount of cell lysate of *Kl*ADH1-engineered cell strains was added and incubated at 4 °C for 60 minutes; the beads were washed with wash buffer; after removal of the wash buffer, a little amount of loading buffer was added and a treatment at 96 °C for 5 minutes was carried out. The above samples were analyzed by polyacrylamide gel electrophoresis.

### Example 8 In vitro protein synthesis system

### 8.1 Preparation of storage solution for the in-vitro protein synthesis system

Final concentration: 22 mM 4-hydroxyethyl piperazine ethanesulfonic acid (pH of 7.4), 30-150 mM potassium acetate, 1.0-5.0 mM magnesium acetate, 1.5-4 mM of a mixture of nucleoside triphosphates (adenosine triphosphate (ATP), guanosine triphosphate (GTP), cytidine triphosphate (CTP) and uridine triphosphate (UTP)), 0.08-0.24 mM of a mixture of amino acids (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine), 25 mM phosphocreatine, 1.7 mM dithiothreitol, 0.27 mg/ml creatine phosphokinase, 0.027-0.054 mg/mL T7 RNA polymerase, 1%-4% PEG, 0.5%-2% sucrose, and finally added 50% by volume of the cell extract.

### 8.2 In vitro protein synthesis reaction

The above-mentioned reaction system was placed in an environment of 20-30 °C, and let the mixture stand for a reaction for 2 to 12 hours.

Assay of the activity of enhanced green fluorescent protein (eGFP): after several hours of reaction, 10 µL of the reaction solution was added into a 96-well white plate or a 384-well white plate; thereafter the mixture was immediately placed in an Envision 2120 multifunctional microplate reader (Perkin Elmer), and the absorbance was read to detect the activity of enhanced green fluorescent protein, where the unit of activity is relative fluorescence unit (RFU) value, as shown in Figure 24.

### Example 9 In vitro protein synthesis and purification

### 9.1 In vitro protein synthesis

In view of the results that, compared with the wild-type cell strain, the ΔADH1 cell strain has significantly reduced transcription and translation activity of cell-free system, while the ADH1 H47K cell strain had no obvious change, accordingly, ADH1 H47K became the material for our *in vitro* protein synthesis study. The cell-free transcription and translation system of the ADH1 H47K cell strain was added with templates of N-His8-eGFP and N-His8-Strep-GFP. The above-mentioned reaction system was placed in an environment of 20-30 °C, and let the mixture stand for a reaction of 2 to 12 hours.

### 9.2 In vitro protein purification

Ni-NTA beads were balanced by binding buffer; an appropriate amount of the above-mentioned reaction system was added and incubated at 4 °C for 60 minutes; the beads were washed with wash buffer; after removal of the wash buffer, a little amount of loading buffer was added and a treatment at 96 °C for 5 minutes was carried out. The above samples were analyzed by polyacrylamide gel electrophoresis. The results are shown in Figure 25.

### The results of the examples of the present invention indicate that:

The results of Example 7 of the present invention showed that, compared with the wild-type cell strain, the affinity to Ni medium generated by the engineered cell strains of the system of the present invention were all significantly reduced.

Among them, cell strains such as ΔADH1, ADH1 H47K, ADH1 H47N, ADH1 H51R, ADH1 H124K, ADH1 H47KH124K and ADH1 H47NH124K had the lowest affinity with Ni medium.

ΔADH1, ADH1 H47K, ADH1 H47N, ADH1 H51R, ADH1 H124K, ADH1 H47KH124K and ADH1 H47NH124K had similar affinity to Ni medium, and the present invention selected ΔADH1 and ADH1 H47K to analyze the transcription and translation activity of cell-free system.

The results were shown in Figure 24. The relative fluorescence unit (RFU) value of transcription and translation activity of the wild-type cell-free system was 442, and the RFU value of ΔADH1 cell-free system was 12, which was significantly reduced than that of the wild-type cell-free system, indicating that the knockout of ADH1 gene had a great negative impact on transcription and translation activity of cell-free system. The relative fluorescence unit (RFU) value of cell-free system of ADH1 H47K was 419, which had no significant change compared with that of the wild-type cell strain.

The same method was used to analyze the cell-free system transcription and translation activity of other mutant cell strains. The results showed that the relative fluorescence unit (RFU) value of cell-free system transcription and translation activity of other mutant cell strains were 300-400 which were substantially equivalent to that of the wild-type cell strain.

In addition, the expression purity of the exogenous proteins N-His8-eGFP and N-His8-Strep-eGFP in the *Kl*ADH1 H47K cell-free transcription and translation system of the present invention was analyzed. The results was shown in Figure 25. Compared with the wild-type cell strains (WT), the *Kl*ADH1 H47K cell strain showed a significantly weakened ADH1 protein band at 37 kD , indicating that the purity of the exogenous proteins N-His8-eGFP and N-His8-Strep-eGFP was significantly improved in the cell-free transcription and translation system of *Kl*ADH1 H47K.

The purity of other mutant cell strains was analyzed by using the same method. The results showed that the purity of the exogenous proteins N-His8-eGFP and N-His8-Strep-eGFP was also significantly improved in the cell-free transcription and translation system of other mutant cell strains compared with that of the wild-type cell strain (WT).

All the above results indicate that the site-directed mutation of ADH family proteins in the present invention can not only reduce the binding ability of ADH family proteins to Ni medium, but also do not affect the cell-free system transcription and translation activity of the strain, and can further effectively improve the expression purity of the exogenous protein in the cell-free system transcription and translation system of the cell strain.

## Claims

1. A cell-free *in vitro* protein translation system or transcription and translation system, comprising a yeast cell lysate of a genetically engineered cell strain, wherein gene encoding its endogenous ADH protein is mutated to a polynucleotide encoding an alcohol dehydrogenase (ADH) mutant protein, wherein
the mutant protein is *Kluyveromyces lactis* ADH1 protein whose amino acid sequence is SEQ ID NO.: 1, wherein the mutant protein has mutations of histidine (H) at one or more positions selected from the following groups: 47, 51, 124 and combinations thereof; or
the mutant protein is *Kluyveromyces lactis* ADH2 protein whose amino acid sequence is SEQ ID NO.: 26, wherein the mutant protein has mutations of histidine (H) at one or more positions selected from the following groups: 45, 49, 122 and combinations thereof, or
the mutant protein is *Kluyveromyces lactis* ADH3 protein whose amino acid sequence is SEQ ID NO.: 27, wherein the mutant protein has mutations of histidine (H) at one or more positions selected from the following groups: 71, 75, 148 and combinations thereof; or
the mutant protein is *Kluyveromyces lactis* ADH4 protein whose amino acid sequence is SEQ ID NO.: 28, wherein the mutant protein has mutations of histidine (H) at one or more positions selected from the following groups: 72, 76, 149 and combinations thereof;
wherein the amino acid sequence of the ADH mutant protein is a polypeptide having at least 99% homology with the sequence shown in SEQ ID No.: 1, 26, 27 or 28,
so that the mutant protein has ADH activity, and compared with the ADH protein in wild-type *Kluyveromyces lactis,* the mutant protein has reduced binding ability to Ni medium.

2. A use of the cell-free *in vitro* protein translation system or transcription and translation system according to claim 1 for *in vitro* protein synthesis, where improved product purity of expressed protein is provided when using Ni medium for purification compared with that of obtained from the wild-type strain.

## Patentansprüche

1. - Zellfreies *in vitro*-Proteintranslationssystem oder -transkriptions- und translationssystem, umfassend ein Hefezelllysat eines gentechnisch veränderten Zellstamms, wobei das Gen, das für sein endogenes ADH-Protein kodiert, zu einer Polynukleotidsequenz mutiert ist, die für ein Mutantenprotein von Alkoholdehydrogenase (ADH) kodiert,
wobei das Mutantenprotein das *Kluyveromyces lactis* ADH1-Protein ist, dessen Aminosäuresequenz SEQ ID NR.: 1 ist, wobei das Mutantenprotein Mutationen von Histidin (H) an einer oder mehreren Positionen aufweist, die ausgewählt sind aus den folgenden Gruppen: 47, 51, 124 und Kombinationen davon; oder
das Mutantenprotein das *Kluyveromyces lactis* ADH2-Protein ist, dessen Aminosäuresequenz SEQ ID NR.: 26 ist, wobei das Mutantenprotein Mutationen von Histidin (H) an einer oder mehreren Positionen aufweist, die ausgewählt sind aus den folgenden Gruppen: 45, 49, 122 und Kombinationen davon; oder
das Mutantenprotein das *Kluyveromyces lactis* ADH3-Protein ist, dessen Aminosäuresequenz SEQ ID NR.: 27 ist, wobei das Mutantenprotein Mutationen von Histidin (H) an einer oder mehreren Positionen aufweist, die ausgewählt sind aus den folgenden Gruppen: 71, 75, 148 und Kombinationen davon; oder
das Mutantenprotein das *Kluyveromyces lactis* ADH4-Protein ist, dessen Aminosäuresequenz SEQ ID NR.: 28 ist, wobei das Mutantenprotein Mutationen von Histidin (H) an einer oder mehreren Positionen aufweist, die ausgewählt sind aus den folgenden Gruppen: 72, 76, 149 und Kombinationen davon;
wobei die Aminosäuresequenz des ADH-Mutantenproteins ein Polypeptid mit mindestens 99 % Homologie zu der in SEQ ID Nr.: 1, 26, 27 oder 28 gezeigten Sequenz ist,
sodass das Mutantenprotein ADH-Aktivität aufweist und im Vergleich zu dem ADH-Protein in dem Wildtyp-*Kluyveromyces lactis* das Mutantenprotein eine verringerte Bindungsfähigkeit an Ni-Medium aufweist.

2. - Verwendung des zellfreien *in vitro-*Proteintranslationssystems oder -transkriptions- und translationssystems nach Anspruch 1 zur *in vitro-*Proteinsynthese, wobei eine verbesserte Produktreinheit des exprimierten Proteins bereitgestellt wird, wenn zum Aufreinigen Ni-Medium verwendet wird, verglichen mit der aus dem Wildtyp-Stamm erhaltenen.

## Revendications

1. - Système de traduction ou système de transcription et de traduction protéique *in vitro* acellulaire, comprenant un lysat de cellules de levure d'une souche cellulaire génétiquement modifiée, le gène codant pour sa protéine ADH endogène étant muté en un polynucléotide codant pour une protéine mutante de l'alcool déshydrogénase (ADH), dans lequel
la protéine mutante est la protéine ADH1 de *Kluyveromyces lactis* dont la séquence d'acides aminés est SEQ ID NO. : 1, la protéine mutante ayant des mutations d'histidine (H) à une ou plusieurs positions choisies dans les groupes suivants : 47, 51, 124 et les combinations de celles-ci ; ou
la protéine mutante est la protéine ADH2 de *Kluyveromyces lactis* dont la séquence d'acides aminés est SEQ ID NO. : 26, la protéine mutante ayant des mutations d'histidine (H) à une ou plusieurs positions choisies dans les groupes suivants : 45, 49, 122 et les combinations de celles-ci ; ou
la protéine mutante est la protéine ADH3 de *Kluyveromyces lactis* dont la séquence d'acides aminés est SEQ ID NO. : 27, la protéine mutante ayant des mutations d'histidine (H) à une ou plusieurs positions choisies dans les groupes suivants : 71, 75, 148 et les combinations de celles-ci ; ou
la protéine mutante est la protéine ADH4 de *Kluyveromyces lactis* dont la séquence d'acides aminés est SEQ ID NO. : 28, la protéine mutante ayant des mutations d'histidine (H) à une ou plusieurs positions choisies dans les groupes suivants : 72, 76, 149 et les combinations de celles-ci ; ou
dans lequel la séquence d'acides aminés de la protéine mutante d'ADH est un polypeptide ayant au moins 99 % d'homologie avec la séquence représentée dans SEQ ID No. : 1, 26, 27 ou 28,
de telle sorte que la protéine mutante a une activité ADH, et par comparaison avec la protéine ADH de type sauvage de *Kluyveromyces lactis*, la protéine mutante a une capacité de liaison réduite au milieu Ni.

2. - Utilisation du système de traduction ou du système de transcription et de traduction protéique *in vitro* acellulaire selon la revendication 1 pour une synthèse protéique *in vitro*, dans laquelle la pureté de produit améliorée de la protéine exprimée est obtenue en utilisant un milieu Ni pour la purification par comparaison avec celle obtenue à partir de la souche de type sauvage.
